(19)

Europäisches
Patentamt
European
Patent Office
Office européen
des brevets

(11) **EP 2 503 009 A1**

(12) **EUROPEAN PATENT APPLICATION**

(43) Date of publication:
**26.09.2012 Bulletin 2012/39**

(51) Int Cl.:
***C12Q 1/68*** *(2006.01)*

(21) Application number: **12425061.4**

(22) Date of filing: **26.03.2012**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME** | (71) Applicant: **Zollo, Massimo**<br>**80131 Napoli (IT)** |
| | (72) Inventor: **Zollo, Massimo**<br>**80131 Napoli (IT)** |
| (30) Priority: **25.03.2011 IT RM20110149** | (74) Representative: **Gitto, Serena et al**<br>**Barzanò & Zanardo Roma S.p.a.**<br>**Via Piemonte, 26**<br>**00187 Roma (IT)** |

(54) **Biomarker for detecting circulating tumor cells and related detecting methods and kit**

(57)    The present invention relates to a biomarker for detecting metastasis of tumor such as colon cancer, esophageal cancer, gastric cancer, breast cancer, lung cancer, medulloblastoma, prostate cancer, lymphoma, melanoma, glioblastoma, pancreas carcinoma, sarcoma, hepatocarcinoma, by means of the detection of copy number and different expression of erbB2 gene in biological fluids of patients with tumors listed above.

**EP 2 503 009 A1**

**Description**

The present invention relates to biomarker for detecting circulating tumor cells and related detecting methods and kit. Particularly, the invention concerns a biomarker for detecting circulating tumor cells such as for instance tumor cells chosen in the group consisting of colon cancer, esophageal cancer, gastric cancer, breast cancer, lung cancer, medulloblastoma, prostate cancer, lymphoma, melanoma, glioblastoma, pancreas carcinoma, sarcoma, hepatocarcinoma. This method is used to determine the copy number variations and the the different expression of erbB2 (from heterogeneous RNA) in circulating tumor cells present in the plasma/serum, peripheral blood, bone marrow, cerebrospinal fluid, ascites fluid, gastric juice, feces, urine, semen, bile, saliva, pleural fluid of patients. Esophageal carcinoma (EC) is the eighth most common cancer and the sixth leading cause of cancer related death worldwide. In European Union, in 2006, were estimated 25.000 cases per years in men and 8.300 in women [1]. In Italy, data from Italian Association for Tumors Registry (AIRTIUM) accounts in 2003, 2.195 cases with 40 new cases per years in Naples (see: http://www.Registri-tumori.it/cms/). The mortality associated with EC is high because tumors are rarely detected before the disease has progressed to an advanced stage. Even when the primary tumor is resectable, the overall 5-year survival rate is under 10% [2]. The stage at which EC is detected is the most important factor in determining prognosis (classified according to the T, N, M system). The rate of lymph node metastasis in both squamous cell carcinoma and adenocarcinoma, the two main histological types of EC, is related to the depth of invasion [3-5]. The majority of ECs that present with symptoms have already invaded the *muscularis propria* (T3) and have already spread to local lymph nodes (N1); this is the reason for the poor prognosis. New approaches to early detection and monitoring of the course of therapy would benefit the clinical management of patients with EC. Several prognostic factors are currently accepted for clinical use, such as nodal status and tumor stage; however, the disease status and clinicopathological conditions cannot unequivocally identify which patients are at low or high risk for disease recur rence [2, 6] . Therefore, there remains the need to identify better prognostic markers that can be used with biological fluids [7, 8].

The human epidermal growth factor receptor (erbB2) oncogene encodes a transmembrane tyrosine kinase receptor that has evolved as a major classifier of invasive breast cancer and a target of therapy for the disease [9]. However, the role of erbB2 in EC is still controversial. Some studies have seen erbB2 overexpression in 20% to 60% of ECs, with this wide range indicating that the differences might depend on tumor stage or histology, or on the interpretation of the immunohistochemistry results. Another study underlined the lack of a prognostic impact of erbB2 amplification in primary ECs [10]. An additionally study showed gene-specific quantitative PCR amplification of the erbB2 gene in tumor cells from lymph nodes and bone marrow from 98 patients with EC [11]. In 50% of 17q12.21 gains, the erbB2 gene was gained in tumor cells from both lymph nodes and bone marrow. Interestingly, only a gain of erbB2 ,and not of 17q12.21, was indicative of poor patient survival, suggesting that erbB2 gains are critical for systemic EC. While a gain in erbB2 in a single disseminated cancer cell has been shown to be an important risk factor in multivariate analysis, erbB2 amplification in primary tumors was not associated with poor survival both in a group of patients that were analyzed for disseminated cancer cells, and in a study cohort comprising twice as many patients [11]. Furthermore, erbB2 gene amplification has been demonstrated in the esophageal adenocarcinoma histotype, in 39 patients versus 39 control samples [12]. The formation of new blood vessels (angiogenesis) and lymph vessels (lymphangiogenesis) significantly contributes to malignant growth and metastasis of solid tumors [13-15]. Angiogenesis and lymphangiogenesis are mediated by distinct cytokines and their receptors. The best characterized and most specific cytokines are the vascular endothelial growth factors (VEGF-A,-B,-C,-D and -E) and their receptors (VEGFR-1,-2 and -3) [16, 17]. Clinicopathological and experimental studies on VEGF-C/D expression in esophageal squamous cell specimens and in esophageal adenocarcinoma specimens have been published [18,19]. Studies on esophageal squamous carcinoma have resulted in a relatively consistent correlation of growth factor expression to tumor progression and lymphatic spread. A recent study reported upregulation of serum VEGF-C in esophageal squamous cell carcinoma, a finding that parallels VEGF-C expression in tissue specimens [20]. They also correlated serum levels of VEGF-C with the presence of lymph node metastasis, and concluded that VEGF-C up-regulation did not a rise from platelets or white blood cells. Many studies have indicated that soluble DNA from tumors can be detected in the serum and plasma of patients with cancers; alterations in both microsatellites and amplification of oncogenes corresponding to the lesions in tumors have been identified in the serum and plasma of patients with several cancers [21-27]. However, a question that remains debatable is whether this DNA is released from primary dissociating tumor cells or from cells that invade the blood. CTCs have been shown to be a critical link between the primary cancers and metastatic disease, which continues to be the leading cause of death for most malignancies [28]. A sensitive and specific system for quantification of CTCs and their free DNA would thus be a useful diagnostic tool in EC, for monitoring the dissemination of tumor cells into the peripheral serum. The authors of the present invention have been found that circulating tumor cells of EC patients have a number of copies of the gene erbB2 greater than healthy cells. In our study we have analyzed erbB2 copy number variations in the free DNA from plasma of EC patients collected before tumors resection and chemotherapy. In particolar, to analyze the copy number variations of genes early markers of tumor progression, we have isolated the DNA of CTCs released in the plasma of patients with esophageal carcinoma. The plasma of 41 patients with EC was collected before their surgical resection and chemotherapy treatment. The data set used had heterogeneous clinical data, consistent with the characteristics of the tumor. Real-time PCR with CN variatons was used for the analysis of DNA from the plasma. Real-time PCR for erbB2 gene showed a significantly copy number variations in plasma of EC patients (p=0.001), with respect to healthy control subjects, with 80% sensibility and a 95% specificity. These variations in erbB2 were significantly negatively correlated to the survival rates of these EC patients (P = 0.03) and appeared to be useful to stratify a subgroup of patients with worse survival rates and low VEGF's expression levels. Therefore, the erbB2 copy number variations from plasma can be used as a prognostic marker for early detection of patients with poor prognosis in esophageal cancer. In clinical practice, the diagnosis and the staging of EC is mainly based on the use of morphological and functional clinical examinations. To date, serum-specific EC markers do not exist, which is in contrast with the situation for other tumors of the gastrointestinal tract. Examples here, can be seen for gastric cancer, where Ca.19.9 has important role in diagnosis. At this time, we can conclude that already identified single biomarkers show a lack of sufficient sensitivity and specificity in EC, and that it is likely that multiple markers will be needed simultaneously to address the diagnosis and prognosis of EC [4].

The relationship between erbB2 overexpression and responses to treatment has not yet been established for EC. New perspectives have been emerging, such as for instance from the TOGA trial, that showed efficacy results from a phase III study of trastuzumab (Herceptin ®), a monoclonal antibody against erbB2: when given with chemotherapy, survival benefits were seen for patients with erbB2-positive early and metastatic gastric cancer [9]. Thus, the data in this TOGA trial showed that trastuzumab with chemotherapy is superior to chemotherapy alone, with median overall survival significantly improved with trastuzumab compared to chemotherapy alone: 13.5 versus 11.1 months, respectively. Trastuzumab is therefore a new and well tolerated treatment for erbB2-positive gastrointestinal cancer [29]. VEGF is an additional marker, which is overexpressed in about 30% to 60% of esophageal tumors. High serum levels of VEGF correlate with advanced stages and poor overall survival in patients that receive curative surgery [30]. Several studies have shown that VEGF-C overexpression correlates with depth of tumor invasion, lym-phatic invasion and lymph node metastasis. CTCs and free DNA in plasma have been analyzed in many tumors, such as breast, colorectal, lung and renal carcinomas. These have been shown to be crucial links between primary cancers and those that reach a metastatic stage, which represent the leading cause of death for most malignancies [31-37].

Our study has evaluated the evolving model of detection of tumor marker CN in DNA from plasma of EC patients. We have here identified erbB2 CN variations in the cell-free DNA of 41 patients with EC in comparison with 34 healthy controls. Our results are in agreement with the study of Chiang PW. et al., that showed erbB2 amplification in 39 plasma from esophageal carcinoma patients with only adenocarcinoma histotype. The system of detection of erbB2 in DNA from plasma by real-time PCR is innovative and with the validation in a larger cohort of patients can be used for monitoring the assessment of the extent of tumor dissemination and the potential development of distant metastasis. This method uses an easier and not invasive peripheral blood sample in respect with the FISH assay (PathVysion FISH assay) performed on tumor biopsies actually approved by the US Food and Drug Administration (FDA) for determining the eligibility for Herceptin clinical treatment in breast carcinoma with erbB2 amplification.

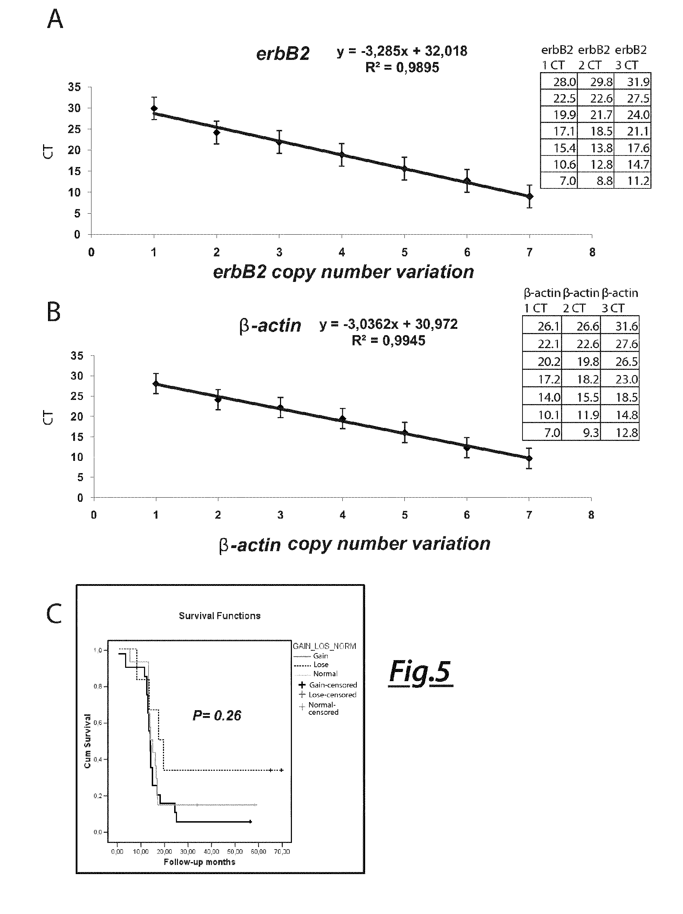

We have further analyzed the erbB2 CN from CTCs isolated from patients with EC and we have demonstrate that it is similar to the DNA isolated from the plasma of the same patients. These data indicate that in the healthy controls, the DNA that we extracted was derived from blood cells (mainly lymphocytes and granulocytes); instead, in the EC patients, the free DNA in the plasma is mainly derived from CTCs (see table 4 for estimation of the DNA content in plasma). At this time, a question remains over the origin of these CTCs: Are those cells derived from primary tumors or from cells that will create a metastases foci? We cannot at present exclude that this DNA derived from the primary tumor cells because of our lack of DNA from the primary tumors in this study. However, our data related to the erbB2 CN, shown for this cohort analysis, is in agreement with an other study that has shown erbB2 amplification in CTCs isolated from lymph nodes and bone marrow in 50% of EC patients [11]. This study demonstrated that the rate of erbB2 amplification is higher in CTCs than in the primary tumors, where the rates of amplification were estimated at about 10% [10, 38]. These data are in agreement with the observation that erbB2 CN variations are a later phenomenon in EC that is mainly associated with the circulating tumor cells. Our data additionally demonstrate that the CTCs isolated from the peripheral blood of these EC patients have a similar type of erbB2 amplification to that seen by Stoecklein et al. (2008) in CTCs isolated from bone marrow and lymph nodes [11]. The present study underlines that the detection of cell-free DNA from plasma through the taking of a non- invasive blood sample can provide relevant information on the disease status of patients with EC. It is important to underline that despite the small number of EC patients (n. 41) included in our cohort, the incidence of this tumor is of 2195 cases for year in Italy with 40 cases in Naples and a raw rate (number of cases per one hundred thousand of in habitants for year) of 2 in southern Italy (data from Italian Association tumors register AIRTUM 2000-2003; see: http://w ww.re gistri-tumori.it/cms/). Furthermore, our dataset is heterogeneous for composition resembling all the clinical characteristics of the esophageal carcinoma. We have shown here a correlation between erbB2 CN from plasma and progression free survival in patients with EC with 70 months (5,8 years) of follow-up. The EC patients were divided into two subgroup on the basis of CN median (cut-off CN = 2), other separation in subgroups were made but they did not shown correlation to progression free survival for the small number of patients here analyzed (see fig. 5). The EC patients with erbB2 CN ≤ 2 showed a better survival with respect to those with CN >2 erbB2. These data demonstrate that the detection of erbB2 CN in the plasma of EC patients can predict the survival rates of patients before the use of clinical resection strategies. These data emphasize the results obtained by Mimura et al., that found an association only between the survival rate and the erbB2 gene amplification (7 positive patients versus 59 negative patients) detected by fluorescence in situ hybridization (FISH) method and not between the survival rate and positive or negative erbB2 staining detected by immunohistochemistry assay [35]. Furthermore, Kuwabara et al. (2009) [39], also demonstrated the correspondence between gene amplification (analyzed by FISH) and protein expression (analyzed by i mmunohistochemistry) of erbB2, underlining that gene amplification is an indicator of poor prognosis in esophageal carcinoma [39].

In particular, we found that an erbB2 CN>2 was correlated to a worst survival rate in the adenocarcinoma histotype. Of note , esophagal adenocarcinoma has been shown to have increased in western countries over the last half century, and especially in European Caucasian white men, although esophageal squamous cell carcinoma remains the dominant type of EC in both western and Asian countries [38, 40, 41]. The rapid increase of this adenocarcinoma histotype has been attributed to an increased prevalence of gastroesophageal reflux disease and obesity [40-42].

Furthermore, our dataset confirms the importance of N status evaluation through our Kaplan-Meier analsis. When divided according to N status, an erbB2 CN >2 impaired survival rates of patients with an N1 status, confirming the importance of the amplification of the erbB2 gene in the prediction of a worse clinical outcome. We also confirmed the importance of the VEGF expression in our dataset, demonstrating that high VEGF expression impairs the survival rate of these EC patients. Moreover, we have identified a new EC category risk: low VEGF expression and erbB2 CN >2. These data are of importance because of the potential new therapies , anti-erbB2, that might be applied to EC patients who will not derive any benefit from anti-V EGF treatment.

The use of these methods, proposed in the following invention, to detect DNA in the plasma of such EC patients should greatly benefit the "early detection " phase before tumor resection and chemotherapy, and should enhance the importance for changes in therapies for those low VEGF-positive EC patients. The validation of the erbB2 detection in a large scale population thus represents a new prognostic marker that can be used as a sign of EC early warning, and for the pre-diction of the disease progression. Thus, inhibition of erbB2 activity might represent a new avenue for successful inhibition of potential metastasis formation, and represents a new therapeutic target for future personalized cancer *therapies.* These data indicate the association between erbB2 CN variations and progression-free survival in these patients with EC.

It is therefore specific object of the present invention an intronic nucleotide sequence of erbB2 gene for use as circulating tumor cells biomarker of a tumor chosen in the group consisting of colon cancer, esophageal cancer, gastric cancer, breast cancer, lung cancer, medulloblastoma, prostate cancer, lymphoma, melanoma, glioblastoma, pancreas carcinoma, sarcoma, hepatocarcinoma, said sequence consisting of:

CGCTGGGCAGGTATGCAgggctgacgtagtgcctttgtggcagcagTTTCGTGG CACACATTCTGC (SEQ ID No: 1) (chr17: 37862429+37862494) or TATGCAGGGCTGACGTAGTGCctttgtggcAGCAGTTTCGTGGCACACA TT (SEQ ID No: 2) (chr17:37862440+37862490) comprised in SEQ ID No:1; or a ribonucleotide sequence corresponding to SEQ ID NO:1 in which T is changed upon with U, i.e.

# CGCUGGGCAGGUAUGCAGGGCUGACGUAGUGCCUUUGUGGCAG CAGUUUCGUGGCACACAUUCUGC (SEQ ID NO: 13).

The present invention also relates to an *in vitro* method for detecting circulating tumor cells of a tumor chosen in the group consisting of colon cancer, esophageal cancer, gastric cancer, breast cancer, lung cancer, medulloblastoma, prostate cancer, lymphoma, melanoma, glioblastoma, pancreas carcinoma, sarcoma, hepatocarcinoma, said method being characterized by the detection of copy number variation of erbB2 gene in cells of a biological sample in comparison to the copy number of said gene in cells of a healthy control by means of TaqMan method using the following primer couple and probe for the amplification of SEQ ID No: 1:

erbB2_for: CGCTGGGCAGGTATGCA (SEQ ID No: 3)
erbB2 _rev : GCAGAATGTGTGCCACGAAA (SEQ ID No: 4)
probe erbB2: CTGACGTAGTGCCTTTGTGGCAGCA (SEQ ID No: 5)
and a primer couple and probe for the amplification of a normalizing gene chosen in the group consisting of: β-actin gene ID: 60, GAPDH Gene ID: 2597, UBB Gene ID: 7314, TUBA6 Gene ID: 84790, RPS13 Gene ID: 6207, NACA Gene ID: 4666, B2M Gene ID: 567, TBP Gene ID: 6908.

Particularly, the primer couple and probe for the amplification of a normalizing gene can consist of the following primer couple and probe for the amplification of β-actin gene:

β-actin _for : CCCAGCCACACCACAAAGTC (SEQ ID No: 6)
β-actin _rev : CCAGTTGAATAAAAGTGCACACCTT(SEQ ID No: 7)
probe β-actin : CACTTGGCCTCATTTT (SEQ ID No: 8)

A further object of the present invention is a method *in vitro* for detecting circulating tumor cells of a tumor chosen in the group consisting of colon cancer, esophageal cancer, gastric cancer, breast cancer, lung cancer, medulloblastoma, prostate cancer, lymphoma, melanoma, glioblastoma, pancreas carcinoma, sarcoma, hepatocarcinoma, said method being characterized by the detection of copy number variations of erbB2 gene in cells of a biological sample in comparison to the copy number of said gene in cells of a healthy control by means of Syber method using the following primer couple for the amplification of SEQ ID No: 2:

erbB2S F: TATGCAGGGCTGACGTAGTGC (SEQ ID No: 9) erbB2 S R: AATGTGTGCCACGAAACTGCT (SEQ ID No: 10)
and a primer couple for the amplification of a normalizing gene chosen in the group consisting of β-actin gene ID: 60, GAPDH Gene ID: 2597, UBB Gene ID: 7314, TUBA6 Gene ID: 84790, RPS13 Gene ID: 6207, NACA Gene ID: 4666, B2M Gene ID: 567, TBP Gene ID: 6908.

Particularly, the primer couple and probe for the amplification of a normalizing gene can consist of the following primer couple and probe for the amplification of β-actin gene:

β-actin F: CCTCACCCTGAAGTACCCCA (SEQ ID No: 11)
β-actin R: TCGTCCCAGTTGGTGACGAT (SEQ ID No: 12)

The present invention also relates to an *in vitro* method for detecting circulating tumor cells of a tumor chosen in the group consisting of colon cancer, esophageal cancer, gastric cancer, breast cancer, lung cancer, medulloblastoma, prostate cancer, lymphoma, melanoma, glioblastoma, pancreas carcinoma, sarcoma, hepatocarcinoma, said method being characterized by the detection of different erbB2 expression in heterogeneous RNA of biological sample cells in comparison to erbB2 expression in heterogeneous RNA of healthy control cells by means of TaqMan method using the following primer couple and probe for the amplification of SEQ ID No: 13:

erbB2 RNA_for: CGCUGGGCAGGUAUGCA (SEQ ID No: 14)
erbB2 RNA rev : GCAGAAUGUGUGCCACGAAA (SEQ ID No:15)
probe erbB2 RNA: CUGACGUAGUGCCUUUGUGGCAGCA (SEQ ID No:16)
and a primer couple and probe for the amplification of a normalizing gene chosen in the group consisting of β-actin gene ID: 60, GAPDH Gene ID: 2597, UBB Gene ID: 7314, TUBA6 Gene ID: 84790, RPS13 Gene ID: 6207, NACA Gene ID: 4666, B2M Gene ID: 567, TBP Gene ID: 6908.

The biological sample can be chosen from plasma/serum, peripheral blood, bone marrow, cerebrospinal fluid, ascites fluid, gastric juice, feces, urine, semen, bile, saliva, pleural fluid of patients.

The methods of the present invention can be advantageously used for the prognosis and diagnosis of cancer.

The present invention also relates to a kit for the *in vitro* detection of circulating tumor cells of a tumor chosen in the group consisting of colon cancer, esophageal cancer, gastric cancer, breast cancer, lung cancer, medulloblastoma, prostate cancer, lymphoma, melanoma, glioblastoma, pancreas carcinoma, sarcoma, hepatocarcinoma, said kit comprising or consisting of the following primers and probes for the detection of copy number variation of erbB2 gene in cells of a biological sample in comparison to the copy number of said gene in cells of a healthy control by means of TaqMan method:

erbB2_for: CGCTGGGCAGGTATGCA (SEQ ID No: 3)
erbB2_rev : GCAGAATGTGTGCCACGAAA (SEQ ID No: 4)
probe erbB2: CTGACGTAGTGCCTTTGTGGCAGCA (SEQ ID No: 5)
and a primer couple and probe for the amplification of a normalizing gene chosen in the group consisting of β-actin gene ID: 60, GAPDH Gene ID: 2597, UBB Gene ID: 7314, TUBA6 Gene ID: 84790, RPS13 Gene ID: 6207, NACA Gene ID: 4666, B2M Gene ID: 567, TBP Gene ID: 6908.

Particularly, the primer couple and probe for the amplification of a normalizing gene can consist of the following primer couple and probe for the amplification of β-actin gene:

> β-actin _for : CCCAGCCACACCACAAAGTC (SEQ ID No: 6)
> β-actin _rev : CCAGTTGAATAAAAGTGCACACCTT(SEQ ID No: 7)
> probe β-actin : CACTTGGCCTCATTTT (SEQ ID No: 8)

In addition, another subject of the present invention is a kit for the *in vitro* detection of circulating tumor cells of a tumor chosen in the group consisting of colon cancer, esophageal cancer, gastric cancer, breast cancer, lung cancer, medulloblastoma, prostate cancer, lymphoma, melanoma, glioblastoma, pancreas carcinoma, sarcoma, hepatocarcinoma, said kit comprising or consisting of the following primers couple for the detection of copy number variation of erbB2 gene in cells of a biological sample in comparison to the copy number of said gene in cells of a healthy control by means of Syber method:

> erbB2 S F: TATGCAGGGCTGACGTAGTGC (SEQ ID No:9)
> erbB2 S R: AATGTGTGCCACGAAACTGCT (SEQ ID No: 10)
> and a primer couple for the amplification of a normalizing gene chosen in the group consisting of β-actin gene ID: 60, GAPDH Gene ID: 2597, UBB Gene ID: 7314, TUBA6 Gene ID: 84790, RPS13 Gene ID: 6207, NACA Gene ID: 4666, B2M Gene ID: 567, TBP Gene ID: 6908. The primer couple and probe for the amplification of a normalizing gene can consist of the following primer couple and probe for the amplification of β-actin gene:
> β-actin F: CCTCACCCTGAAGTACCCCA (SEQ ID No: 11)
> β-actin R: TCGTCCCAGTTGGTGACGAT (SEQ ID No: 12)

The present invention also concerns a kit for *in vitro* detection of circulating tumor cells of a tumor chosen in the group consisting of colon cancer, esophageal cancer, gastric cancer, breast cancer, lung cancer, medulloblastoma, prostate cancer, lymphoma, melanoma, glioblastoma, pancreas carcinoma, sarcoma, hepatocarcinoma, said kit comprising or consisting of the following primer couple and probe for the detection of different erbB2 expression in heterogeneous RNA of biological sample cells in comparison to erbB2 expression in heterogeneous RNA of healthy control cells by means of TaqMan method:

> erbB2 RNA_for: CGCUGGGCAGGUAUGCA (SEQ ID No: 14)
> erbB2 RNA rev : GCAGAAUGUGUGCCACGAAA (SEQ ID No:15) probe erbB2 RNA: CUGACGUAGUGCCUUUGUGGCAGCA (SEQ ID No:16)
> and a primer couple and probe for the amplification of a normalizing gene chosen in the group consisting of β-actin gene ID: 60, GAPDH Gene ID: 2597, UBB Gene ID: 7314, TUBA6 Gene ID: 84790, RPS13 Gene ID: 6207, NACA Gene ID: 4666, B2M Gene ID: 567, TBP Gene ID: 6908.

Another object of the present invention is an intronic nucleotide sequence of erbB2 gene consisting of:

> CGCTGGGCAGGTATGCAgggctgacgtagtgcctttgtggcagcagTTTCGTGG CACACATTCTGC (SEQ ID No: 1) or
> TATGCAGGGCTGACGTAGTGCctttgtggcAGCAGTTTCGTGGCACACA TT (SEQ ID No: 2) or a ribonucleotide sequence corresponding to SEQ ID NO:1 in which T is changed upon with U, i.e.
> GCUGGGCAGGUAUGCAGGGCUGACGUAGUGCCUUUGUGGCAGC        AGUUUCGUGGCACACAUUCUGC (SEQ ID NO: 13).

The present invention will now be described for illustrative but not limitative purposes, according to preferred embodiments, with particular reference to the figures of the accompanying drawings:

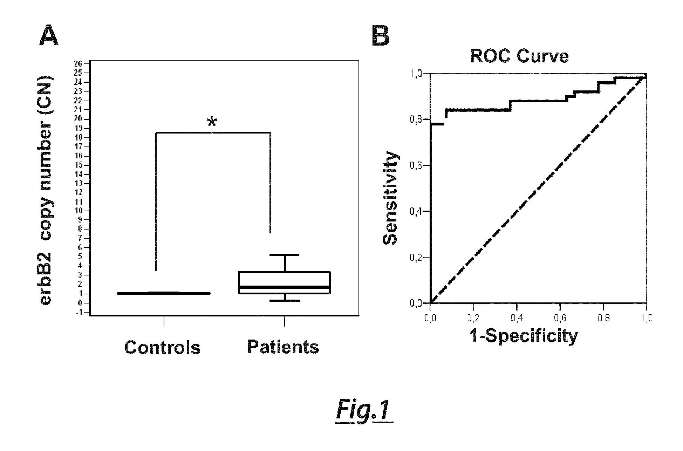

> **Figure 1 shows ErbB2 copy number variations in DNA in plasma from patients with esophageal carcinoma versus healthy controls and relative ROC curve analysis of the assay** (A) Box plot showing the ranges of erbB2 CN in DNA from the plasma of patients with EC (n.41) with respect to healthy controls (n.34) *$P$ = 0.001. Horizontal bars in the box represent the medians of CN which is equal to 2 for patients and 1 for controls (B) ROC curves (solid line; AUC, 0.95) were generated to test the specificity and sensitivity of *erbB2* CN (80% sensitivity e 95% specificity (ashed line; AUC, 0.5; P = 1.39 $\times$ 10$^{-11}$). Data are representative of three independent experiments.

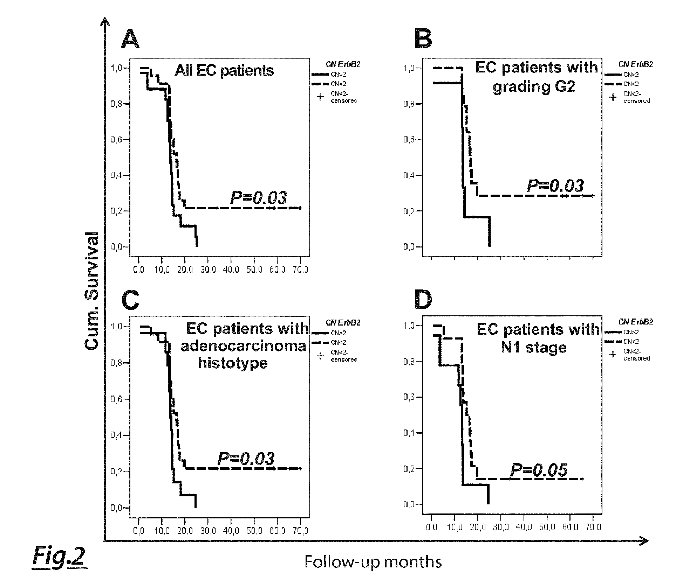

> **Figure 2 shows Kaplan-Meier survival curves for erbB2 copy number variations in plasma from patients with esophageal carcinoma according to clinical characteristics** (A) All patients with EC; (B) EC patients with tumor grading G2; (C) EC patients with adenocarcinoma histology; (D) EC patients with N1 stage disease. The patients are divided according to erbB2 CN≤2 (solid line) and erbB2 CN >2 (dashed line). Data are representative of three independent experiments

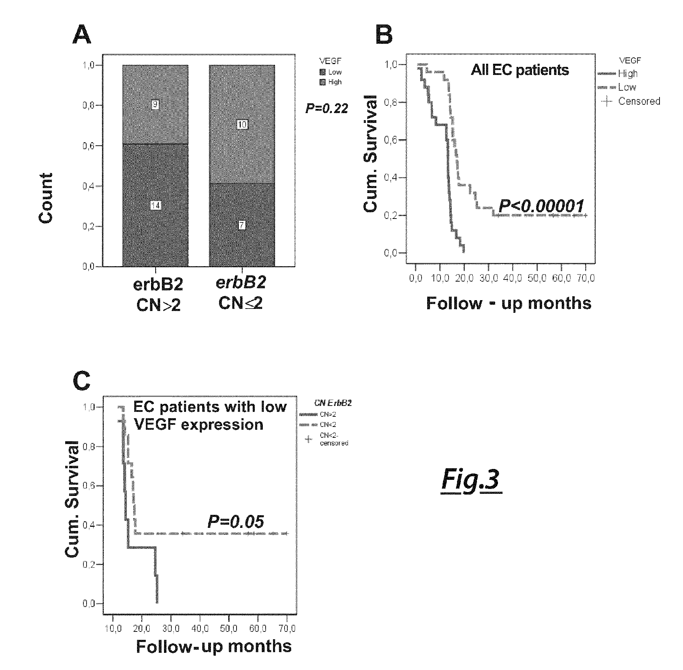

**Figure 3 shows ErbB2 copy number variations in plasma from patients with esophageal carcinoma according to VEGF plasma levels.** (A) Histogram showing lack of significant correlation between erbB2 CN variations and VEGF plasma levels in all patients with EC (n. 41), as indicated. The numbers in the bars show the EC patients in each sub-group (B, C). Kaplan-Meier survival curves for all patients with EC according to low and high plasma levels of VEGF (B), and for patients with EC and low plasma levels of VEGF according to erbB2 CN≤2 (blu line) e *erbB2* CN >2 (green line). Data are representative of three independent experiments.

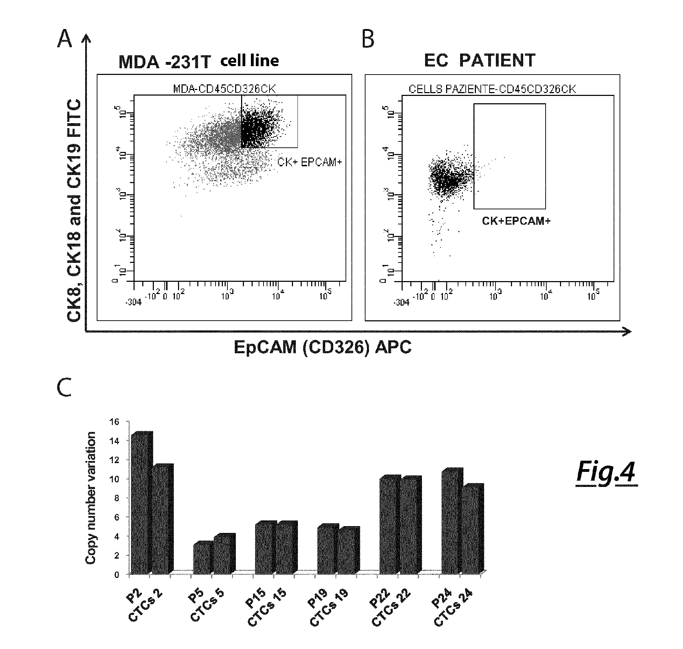

**Figure 4 shows Isolation and separation of CTCs using "fluorescence -activated cell sorting" FACS (A, B)** Scatter plots for gating of cells positive to CK8, CK18 , CK19 and EpCAM in the MDA-231T breast cancer cell line (A; positive control) and from the blood of a representative patient with EC (B) **(C)** Histogram showing erbB2 CN variations from sorting CTCs of patients with EC compared to the CN of DNA from plasma of the same patients. Data are representative of three independent experiments.

**Figure 5 shows calibration curve for erbB2 and β-actin genes relating cycle threshold CT and gene copy number (A)** For the erbB2 gene CN. Top right, equation of the curve and the relative mean correlation coefficient ($R^2$). **(B)** For the β -actin gene CN. Top right, as for (A). **(C)** Kaplan-Meier survival curves for all patients with EC according to lose, normal and gain erbB2 CN.

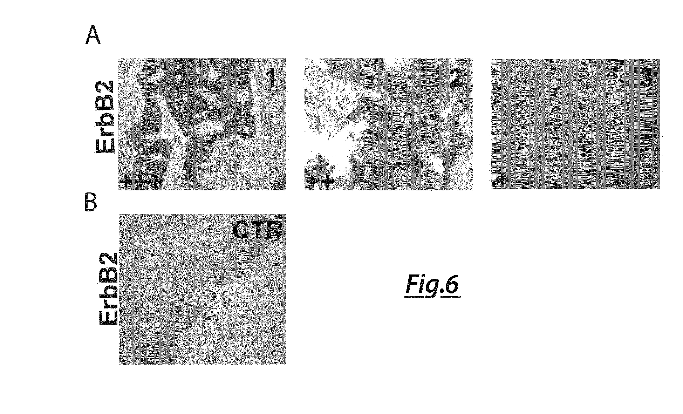

**Figure 6 shows ErbB2 in esophageal tumor tissues. (A)** Patterns of ErbB2 staining with a polyclonal antibody in EC tumor tissues 1, 2 and 3, showing scores of +++, ++ and +, respectively. **(B)** ErbB2 staining as for (A), in healthy esophageal mucosa (CTR)

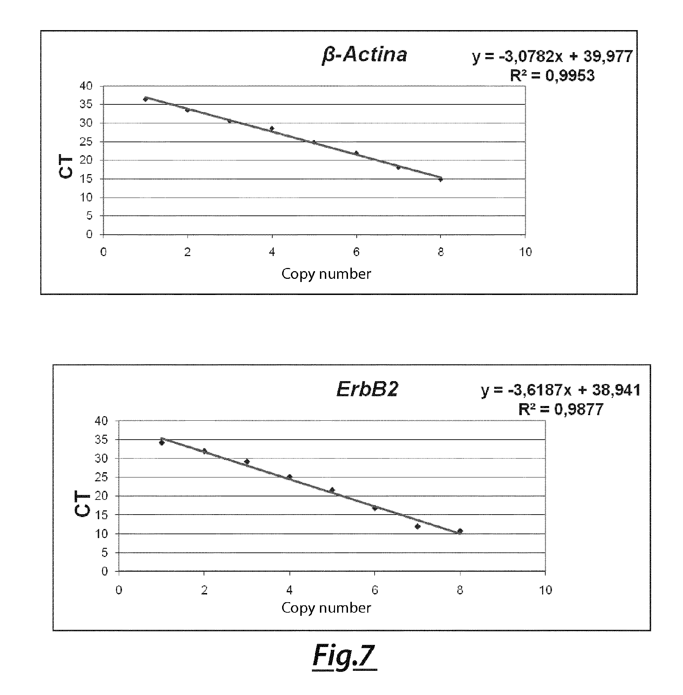

**Figure 7 shows calibration curve for erbB2 and β-actin genes relating cycle threshold CT and gene copy number.** On top for β-actin gene equation of the curve and the relative mean correlation coefficient ($R^2$). At the bottom for erbB2 gene.

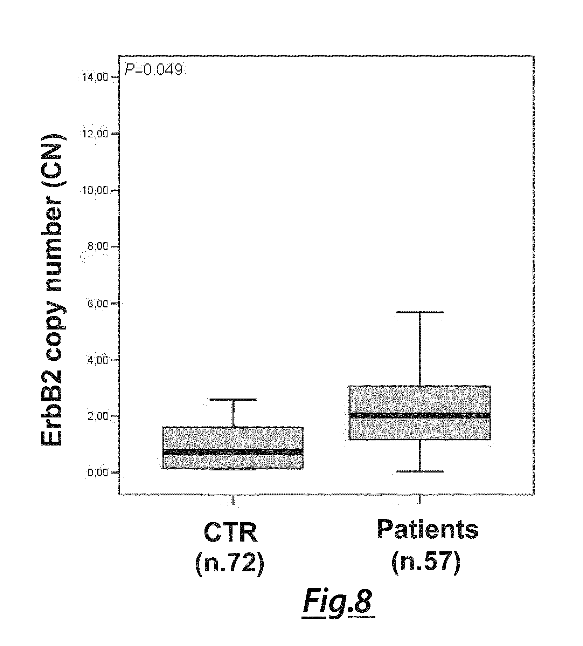

**Figure 8 shows ErbB2 copy number variations in plasma from patients with gastric carcinoma versus versus healthy controls.** Box plot showing the ranges of erbB2 CN in DNA from the plasma of patients with EC (n.57) with respect to healthy controls (n.72) *$P$ = 0.04. Horizontal bars in the box represent the medians of CN

**Example 1:** Detection of erbB2 copy number variations in plasma of patients with esophageal carcinoma

**Materials and Methods**

**Sample collection**

The present study included 41 patients (men and women; aged 52-74 years) with EC, as diagnosed by the Dipartimento Medico-Chirurgico di Internistica Clinica e Sperimentale" F. Magrassi e A. Lanzara" SUN, Seconda Università degli Studi, Naples, Italy. The ethics committee of the SUN approved this study, and informed written consent was obtained f rom all of the patients before inclusion in the study.

Table 1 shows the clinicopathological characteristics of the esophageal carcinoma patients (EC).

Table 1

| Features | Number of patients (%) | *erbB2* copy number (CN) | | *P* |
|---|---|---|---|---|
| | | ≤2 (%) | >2 (%) | |
| **Age (years)** | | | | |
| **>60** | 9 (22) | 5 (20.8) | 4 (23.5) | 0.84 |
| **≤60** | 32 (78) | 19 (79.2) | 13 (76.5) | |
| **T stage** | | | | |
| **T1** | 0 (0) | | | |
| **T2** | 8 (20) | 4 (16.7) | 4 (25) | 0.81 |
| **T3** | 27 (67.5) | 17 (70.8) | 10 (62.5) | |
| **T4** | 5 (12.5) | 3 (12.5) | 2 (12.5) | |
| **N stage** | | | | |
| **N0** | 15 (39.5) | 9 (39.1) | 6 (40) | 0.96 |

(continued)

| | | | | |
|---|---|---|---|---|
| **N stage** | | | | |
| **N1** | 23 (60.5) | 14 (60.9) | 9 (60) | |
| **N2** | 2 (3.4) | ND | ND | |
| **Tumor grading** | | | | |
| **G1** | 9 (24.3) | 5 (22.7) | 4 (11.1) | 0.28 |
| **G2** | 20 (54.1) | 14 (63.6) | 6 (40) | |
| **G3** | 3 (13.6) | 5 (33.3) | 8 (21.6) | |
| **Tumor location** | | | | |
| **upper** | 7 (17.1) | 6 (25) | 1 (5.9) | 0.05 |
| **middle** | 20 (48.8) | 8 (33.3) | 12 (70.6) | |
| **lower** | 14 (34.1) | 10 (41.7) | 4 (23.5) | |
| **Histology** | | | | |
| **squamous** | 26 (63.4) | 13 (54.2) | 13 (76.5) | 0.14 |
| **adenocarcinoma** | 15 (36.6) | 11 (45.8) | 4 (23.5) | |

T and N, the clinical stages evaluated according to the UICC staging criteria.

Thirty-four peripheral EDTA blood samples from 34 healthy volunteers formed the negative control group. The peripheral blood of both cases and controls was centrifuged at 2850 $\times$ g for 10 min at 4°C, and the supernatant (plasma) was store d at -80°C until analysis. The time lag between blood collection and plasma processing was at most of four hours for both EC patients and healthy controls. All of the plasma samples from the patients with EC included in the present study were collected before surgical tumor resection and before chemotherapy treatment

### DNA preparation from plasma

A total of 500 µL of plasma was treated with 1 mg mL$^{-1}$ proteinase K (GIBCO) and 10% SDS (Sigma Aldrich) for 1 h at 65°C. These samples were then heated to 95°C for 10 min, to inactivate the proteinase K. The DNA in the samples was then phenol extracted and ethanol precipitated. After centrifugation at 6000 $\times$ g for 15 min at 4°C, the DN A pellets were dissolved in 30 µl sterile water

### Cloning of the real-time PCR amplification products of erbB2 and β-actin for copy number analysis

Following PCR amplification, the real-time PCR amplification products for erbB2 and β-actin were cloned separately into the pcr 2.1 vector, using the original TA cloning system (Invitrogen). Briefly, fragments of the erbB2 and β-actin genes were amplified from genomic DNA of healthy control peripheral blood. The PCR primers and cycling conditions were the same as those used for real-time PCR of the DNA from the EC patients. After the cloning into the pcr 2.1 vector, the erbB2 and β-actin plasmids were prepared at a concen-tration of 100 ng/ µl.

### Determination of erbB2 copy number variations

### Real-time PCR

Real-time quantitative PCR (Syber Green method) was performed using standard protocols with an Applied Biosystems ABI PRIS M7900HT Sequence Detection system. Briefly, 100 ng DNA was added to 12.5 µl of SYBER-green PCR master mix (Applied Biosystems), with 600 nM of each primer, and water to 25 µl. The reactions were amplified with a single step of 2 min at 50°C, 5 min at 95° C, and then for 40 cycles of 5 s at 95° C, and 1 min a t 60°C. The thermal denaturation protocol was run at the end of the PCR to determine the number of products that were present in the reactions. All of the reactions were run in triplicate and included non template controls for each gene. The amount of each gene was normalized to β-actin as the reference gene. The real-time PCR primers for each gene were designed using the Primer Express software, version 2.0 (Applied Biosystems), with a Tm of 60°C and a primer length of between 18 nt and 25 nt. We used a standard analysis to calculate the amplification of the erbB2 genes by th e 2^- DCt method as described previously [43]. The real-time PCR was performed twice for each sample, and we used the mean value of these two independently data points.

**Calibration curves**

For each assay, we prepared a reference calibration curve as described previously, [44] which containing a nine-concentration titration series representing the erbB2 and β-actin genes at 10-fold dilutions from 1.0 ng/μl a 1.0 × $10^{-8}$ ng/μl. Each calibration curve was produced in triplicate. We tested the reproducibility of the calibration curves for erbB2 and β-actin genes determination according to the slopes and the correlation coefficients of the experimental fittings of the calibration curves from each experiment. The mean slopes of the calibration curves for the two genes were similar: -3.285 for erbB2 and - 3.036 for β-actin. The mean correlation coefficients of the curve fitting were 0.989 and 0.994, respectively. We here, showed three representative slopes for β-actin and erbB2 with the standard errors: slopes b-actin: - 3.122 ; - 2.772; - 3 215, standard deviation is 0. 234 and standard error is 0.135; slopes erbB2:-3,263; -3,232; -3,360, standard deviation is 0,066 and standard error 0. 039 . A slope of -3.3 +/ - 10% reflects an efficiency of 100% +/- 10% of the PCR reaction [45]. For this reason, the slopes for the two genes are similar (p values = 0 ,150). We calculates these gene CN of the patients using the Ct values of the vector and calculating the CN vector according to the formula [44, 46, 47]:

$$\text{Number of copies= (amount (ng) * 6.022} \times 10^{23}) / (\text{length (bp) * 1.0} \times 10^{9} * 650).$$

This formula takes into account 6.0 22 × 10 $^{23}$ (molecules/mole) that is the Avogadro' s number and 660Da, that is the average weight of a single base pair.

The non-integer results were treated on the basis of the cut-off CN >2 and CN ≤ 2 and they were rounded off, particularly, the values were rounded down if the decimal value was between 0 and 4; while they were rounded up if the decimal value was between 5 and 9.

The primers for the erbB2 gene were:

*erbB2* F: TATGCAGGGCTGACGTAGTGC (SEQ ID No: 9)
*erbB2* R: AATGTGTGCCACGAAACTGCT (SEQ ID NO: 10)

The primers for the b -actin gene were:

β-actin F: CCTCACCCTGAAGTACCCCA (SEQ ID NO: 11)
β-actin R: TCGTCCCAGTTGGTGACGAT (SEQ ID NO: 12)

**Real time PCR TaqMan method**

The real time TaqMan Singloplex and Multiplex PCR was performed with the same Applied Biosystems ABI PRISM 7900HT Sequence Detection System used for the real time Syber Green method. The reactions was performed in a final volume of 20 μl. 100 ng DNA was added to 10 μl of master mix TaqMan Universal 2.0 (Applied Biosystems) in the singolplex method or to 10 μl di master mix TaqMan Environmental 2.0 (Applied Biosystems) in the multiplex method. To the mix were added also 0,3 μM of each primer, the probe and water until to the final volume of 20 μL. The reactions were incubated in a 96-well plate at the following temperatures: 2 min at 50 °C, 5 min at 95 °C, followed by 40 cycles of 5s at 95 °C, and 1min a 60°C. The TaqMan Universal master mix contains: DNA polymerase AmpliTaq Gold, AmpErase UNG enzyme, i dNTPs, the passive reference 1 and saline buffer with magnesium chloride. The enzyme DNA polymerase AmpliTaq Gold is activated only when it reaches the temperature of 95 °C and it allows to obtain high performance without the formation of nonspecific products, including dimers of primers. The passive reference 1 is used to normalize the signal emetted by the probes and to minimize the variability due to various causes such as evaporation of the sample. The enzyme AmpErase UNG prevents the reamplification of dU-containing PCR products resulting from contaminants such as RNA. The TaqMan Environmental 2.0 master mix is specific for multiplex real time and contains DNA polymerase AmpliTaq Gold and the passive reference. The stability was tested at 37 ° C for more than 5 weeks.

The primer couple and probe for the erbB2 gene amplification are:

erbB2_for: CGCTGGGCAGGTATGCA (SEQ ID NO: 3)
erbB2_rev : GCAGAATGTGTGCCACGAAA (SEQ ID NO: 4)
probe erbB2: CTGACGTAGTGCCTTTGTGGCAGCA (SEQ ID NO: 5)

The primer couple and probe used for normalization are:

β-actin _for : CCCAGCCACACCACAAAGTC (SEQ ID NO: 6)
β-actin _rev : CCAGTTGAATAAAAGTGCACACCTT(SEQ ID NO: 7)
probe β-actin : CACTTGGCCTCATTTT (SEQ ID NO: 8)

**Cell sorting of circulating tumor cells by flow cytometry**

Whole peripheral blood samples from six EC patients were centrifuged at 2850 × g for 10 min at 4° C, and the plasma was removed. The resulting cell pellet was suspended by dilution with phosphate buffered saline (PBS) and then centrifuged at 1700 × g for 30 min at 4°C, to separate mononuclear cells. Then, the mononuclear cells were stained at 4°C and avoiding light exposure with: cytokeratins CK8-, CK18-, and CK19-FITC (mono-clonal antibody A45-B/B3; Micromet, Munich, German y) 1:3 0; CD3 26-A PC (EpCAM, Becton Dickinson) 1:10; and CD45-PerCP (Be cton D ic kinson) 1:10.; and CD45-PerCP (BECton Dickinson) 1:10. The samples were then washed with 2 ml PBS with 2% fetal bovine serum, and centrifuged at 800 × g for 3 min at 4° C. The supernatant was removed and the cell pellets were suspended as before (2 ml PBS with 2% fetal bovine serum), and filtered with 30 μm filcons (Becton Dickinson) before analysis by flowcytometry. The flowcytometry gating strategies included: a first gate based on physical parameters forward scatter (FSC) versus side scatter (SSC), to eliminate cell debris and dead cells. The leukocyte population was excluded with a second gate in the CD45 versus SSC dot plot. The CTCs were selected by a gate based on cytokeratin CK8, CK18 and CK 19/CD 326- positive cells. These cells were purified by cell sorting with a BD FACS Aria instrument, and collected in a 2 ml tube.

**Immunohistochemistry**

One or two representative tumor blocks from each patient were examined by immunohistochemistry. We analyzed erbB2 for 14 EC tissue samples with an anti-erbB2 anti-body (1:5 0 0; Dako Cytomation). The unmasking was performed in 10 mM citrate buffer, pH 6, for 45 min at 97°C. Blocking was performed with BSA 2 ,5%, normal goat serum 0.05%, PBS 1%, Tween 0,5% for 1 h at room temperature. The signal was revealed according to DAKO kits, for 15 min (each, for biotin and streptavidin), at room temperature. DAB was from Dako Cytomation, and the slides were mounted and examined under a Leica DC50 0 compound microscope (Nussloch, Germany). ErbB2 immunohistochemical staining in EC samples was scored according to Kuwabara et al.,[39], who adopted the same criteria previously validated for breast cancer; namely: no staining or weak staining in fewer than 10 % of the tumor cells (-); weak staining in part of the membrane in more than 10 % of the tumor cells (+); complete staining in part of the membrane with weak or moderate intensity in more than 10% of the tumor cells, (++); strong staining in more than 10% of the tumor cells, (+++).

**VEGF ELISA assay**

The levels of the VEGF protein in the plasma used f or er bB2 amplifications from 41 EC patients were determined using a commercially available sandwich enzyme immunoassay kit (Endogen VEGF ELISA kit, Cambridge, USA). All sample were assayed in duplicate.

**Statistical analysis**

All of the data are presented as medians ± standard error. Statistical significance was calculated using the Mann-Whitney test. Raw real-time PCR data for erbB2 amplification in the plasma from healthy controls and patients with EC were normalized using the β-actin amplification values. The erbB2 CN values were divided into two groups, as " CN ≥ 2 " and " CN <2" Potential associations between clinicopathological variables and erbB2 CN were analyzed by Pearson's Chi-Squared. Kaplan-Meier survival curves were constructed for erbB2 and VEGF low and high level groups, as well as for the clinicopathological variables available in our dataset. Differences in survival between the groups were tested for statistical significance by log-rank tests. Progression free survival time was measured from the date of registration in the study to the date of progression or last follow-up visit. The patients derived all from one clinic and were all of Caucasian origin. P <0.05 was considered as statistically significant. Receiver operating characteristic (ROC) curves and the area under the ROC curves were generated to calculate the specificity and sensitivity of erbB2 CN variation in the healthy controls and the EC patients.

**Results**

**Detection of erbB2 copy number variations in DNA from plasma of patients with esophageal carcinoma**

Real-time PCR with CN analysis was used for the analysis of DNA from the plasma of 41 patients with EC, before their surgical resection and chemotherapy treatment. Here, we also analyzed the CN variations for erbB2 in 34 healthy controls. As shown in Figure 1A, the erbB2 CNs were significantly higher in the plasma samples from the 41 EC patients, with respect to the 34 healthy control subjects (P=0.001). In particular, 24 EC patients had CN ≤ 2 (58.5%), while 17 EC patients had CN >2 (41.4%) with a median CN of 2 and a standard deviation of 5.02. The 34 healthy control subjects showed a median of erbB2 CN of 1, with a standard deviation of 0.16. The representative calibration curves for the erbB2 and β-actin genes used for the CN variation analysis are shown in Fig. 5.

We also evaluated the CN data of erbB2 by real-time PCR using the ROC curves, which provide an analysis of the sensitivity and specificity of the assay. The ROC curves were constructed for the erbB2 CN in plasma, examining the 41 EC patiets and 34 healthy controls. As shown in Figure 1B, in this sample set, erbB2 had an area under the curve (AUC) of 0.9 5, and differed significantly from that of a chance result (AUC: 0.5) (P =1.39 ×10 $^{-11}$). For distinguishing patients with EC from healthy controls, erbB2 had an 80% sensibility and a 95% specificity. The association of the erbB2 CN variations with the clinicopathological characteristics of the EC patients are summarized in table 1. The a ssociation of erbB2 CN and the clinical fea-tures of the esophageal carcinoma were also assessed by the Chi-Squared Test. There were no significant statistical direct correlations between erbB2 CN and these clinical features, except for tumor location when associated with erbB2 CN >2 (P = 0.05). Despite the small number of patients, our dataset comprises all the characteristic of the tumor as regard T stage, N status, tumor grading , tumor location and histology (tab. 1). All CT values of real time PCR assay for EC patients and healthy controls subjects were showed in the table 2

Table 2

| EC patients code | CT β-actin | CT β-actin | Healthy subjects code | CT erbB2 | CT β-actin |
|---|---|---|---|---|---|
| P1 | 21.94 | 20.46 | e10 | 31.54 | 26.63 |
| P2 | 25.97 | 26.01 | e12 | 25.13 | 20.16 |
| P3 | 31.77 | 26.45 | e13 | 28.62 | 24.28 |
| P4 | 23.54 | 22.50 | e14 | 29.46 | 24.66 |
| P5 | 28.54 | 27.04 | e2 | 34.69 | 29.60 |
| P6 | 26.84 | 27.55 | e20 | 31.69 | 26.91 |
| P7 | 26.80 | 25.02 | e22 | 29.20 | 24.88 |
| P8 | 23.70 | 22.45 | e23 | 27.59 | 22.78 |
| P9 | 23.28 | 21.80 | e3 | 28.53 | 23.72 |
| P10 | 28.98 | 27.29 | e40 | 26.75 | 23.63 |
| P11 | 21.79 | 20.40 | e41 | 23.36 | 20.97 |
| P12 | 29.51 | 27.62 | e46 | 29.43 | 26.72 |
| P13 | 26.50 | 25.38 | e48 | 27.99 | 24.96 |
| P14 | 28.57 | 26.85 | e49 | 28.58 | 23.38 |
| P15 | 27.71 | 26.64 | e5 | 31.21 | 25.92 |
| P16 | 22.51 | 21.11 | e51 | 29.03 | 23.99 |
| P17 | 26.57 | 25.02 | e52 | 29.73 | 24.83 |
| P18 | 21.80 | 20.64 | e53 | 27.69 | 22.62 |
| P19 | 27.50 | 26.48 | e55 | 30.00 | 24.88 |
| P20 | 26.43 | 25.27 | e56 | 27.81 | 22.51 |
| P22 | 37.16 | 26.94 | e57 | 30.34 | 24.94 |
| P24 | 22.98 | 21.16 | e6 | 29.04 | 23.84 |
| P25 | 24.21 | 21.98 | e7 | 30.16 | 24.79 |
| P26 | 24.28 | 22.87 | e8 | 29.54 | 24.13 |
| P27 | 24.73 | 22.87 | f3 | 24.09 | 20.37 |

(continued)

| EC patients code | CT erbB2 | CT β-actin | Healthy subjects code | CT erbB2 | CT β-actin |
|---|---|---|---|---|---|
| P28 | 30.16 | 27.35 | f4 | 23.01 | 19.44 |
| P29 | 27.63 | 25.78 | f5 | 25.96 | 22.33 |
| P30 | 23.69 | 21.59 | f6 | 24.27 | 20.57 |
| P31 | 23.45 | 20.87 | e1 | 28.55 | 24.01 |
| P32 | 21.78 | 19.34 | e15 | 31.79 | 27.47 |
| P33 | 21.47 | 19.20 | e18 | 29.67 | 25.63 |
| P34 | 21.76 | 20.40 | e19 | 30.80 | 26.29 |
| P35 | 20.91 | 18.53 | e21 | 27.92 | 23.20 |
| P36 | 21.78 | 21.38 | e24 | 32.45 | 27.92 |
| P37 | 25.14 | 25.61 | | | |
| P39 | 23.25 | 20.52 | | | |
| P40 | 27.66 | 25.16 | | | |
| P41 | 21.93 | 19.55 | | | |
| P42 | 28.12 | 26.29 | | | |
| P43 | 31.68 | 26.43 | | | |
| P44 | 22.32 | 19.56 | | | |

The table 3 shows copy number variations (CN) of CTCs from EC patients compared to copy number variations (CN) of DNA from plasma of the same patients

Table 3

| CN patients code | Copy number variation of erbB2 | CTCs patients | Copy number variation of erbB2 |
|---|---|---|---|
| P2 | 15 | CTCs 2 | 11 |
| P5 | 3 | CTCs 5 | 4 |
| P15 | 5 | CTCs 15 | 5 |
| P19 | 5 | CTCs 19 | 5 |
| P22 | 10 | CTCs 22 | 10 |
| P24 | 11 | CTCs 24 | 9 |

ErbB2 copy number variations was performed also with TaqMan method (TaqMan Multiplex PCR, see material and methods) in 39 DNA extracted from plasma of patients with esophageal carcinoma included among those previously analyzed. Figure 7 shows calibration curves for *erbB2 and* β-*actina* genes. As shown in table 5, in these patients the erbB2 CN is >2 in the 79.99% of the cases.

Table 5

| Copy number | ErbB2 |
|---|---|
| ≤2 | 8 (20.51%) |
| >2 | 31 (79,49%) |
| Patients number | 39 (100%) |

ErbB2 copy number variations were performed with TaqMan method also in patients with gastric carcinoma. The real time PCR (Taqman) for copy number variations was performed using DNA extracted from plasma of 57 patients with gastric carcinoma, before surgical tumor resection and chemotherapy treatment. We also analyzed the erbB2 CN in 72 healthy control. As shown in figure 8, erbB2 CN were significantly higher in samples of plasma from patients with gastric carcinoma compared to healthy controls (P=0.04).

**ErbB2 copy number variations predict survival rates in esophageal carcinoma patients**

We used Kaplan- Meier analyses to look for correlations between the erbB2 CN variations and EC patient progression free survival. The survival curves for the EC patients were divided according to erbB2 CN ≤ 2and erbB2 CN>2, as illustrated in Figure 2 on the basis of CN median of EC patient s. We also tried to divide the patients into lose (CN <1), normal (2 ≥ CN ≥ 1) and gain (C N≥ 2) of erbB2, 6 , 14 and 21 patients respectively, and by using Kaplan-Meier analyses we did not reach a statistical significance but we saw the same tendency (fig. 2) as for the survival analysis with cut-off 2 (Fig. 5C). The number of patients here analyzed is too small to be divided into three groups, for this reason we analyzed all the data with the CN cut-off value of 2. ErbB2 CN >2 was signficantly negatively correlated to the survival rates of these EC patients (P = 0.03; Figure 2A). In further analyses according to the tumor grading, the EC patients with a tumor grading G2 can be further stratified according to erbB2 CN ≤ 2 and erbB2 CN >2, which were a gain significantly negatively correlated to the survival rates of these EC patients (P = 0.03; Figure 2B). These analyses for the EC patients with tumor gradings G1 and G3 did not show any statistically significant effects on their survival rates due to the small numbers of patients in these subgroups (data not shown; see table 1). Our dataset also showed an heterogeneous histological composition when stratified accordingly to the histotype. Here, erbB2 CN variations were significantly correlated to the survival rates of the adenocarcinoma cases (P = 0.03; Figure 2C). Similarly, we analyzed the correlations between the N status and survival rates: erbB2 CN >2 in the N1 subgroup was significantly negatively correlated to the survival rates of these EC patients (P =0.05, Fig. 2D). Again, these analysis for the EC patients with n N status of N2 and N3 did not show any statistical significance for the survival rates due to the small numbers of patients in these subgroups (data not shown; see table 1).

**Correlations of erbB2 copy number variations with VEGF plasma levels**

We then tested erbB2 CN for possible associations with VEGF protein levels in the plasma from the same EC patients (Figure 3). The VEGF levels data were available in our tissue/serum databank collection , and have been previously published [48]. The erbB2 CN variations did not show any significant direct associations with VEGF levels in the plasma (Figure 3A). However, when the EC patients were stratified into the low and high VEGF groups, as shown in Figure 3B, high VEGF levels in the plasma of those EC patients were significantly negatively correlated to their survival rates (P < 0 .00001). In a further Kaplan-Meier analysis, we also looked at the influence of erbB2 CN variations in these EC patients with low and high VEGF levels: an erbB2 CN >2 was significantly negatively correleted to the survival rate of the EC patients with low VEGF expression (P=0.05; Figure 3C). Altogether, these results show that erbB2 CN >2 status and low VEGF levels in the plasma of these EC patients appear to be useful to stratify a subgroup of patients with worse survival rates.

**Isolation of circulating tumor cells from peripheral blood of esophageal carcinoma patients**

To answer the question of whether this DNA in the plasma of these EC patients was derived from CTCs, we isolated these cells from these patients. Indeed, we detected CTCs that were disseminated in the peripheral blood in six of these patients with EC. The CTCs were isolated from the peripheral blood of these EC patients by sorting the cells negatively for CD45 (a specific antigen for lymphocytes) and by a gate based on epithelial cytoskelet on components: the cytokeratins CK8, CK18 and CK19, and CD326 (EpCAM) (see Methods). Both, the cytokeratins (CK8, CK1 8 and CK19) and the antigen EpCAM have been used to identified epithelial cell adhesion molecules, as previously used by Stoecklein et al. as markers to isolate CTCs from lymph nodes and bone marrow of patients with EC [11]. As previously described [49], we used the MDA-231T breast cell line as the positive control (Figure 4A). Here we show that we were only able to successfully isolated positive cells from the blood samples from the EC patients (about 0.05 %-0.1% of the $2 .0 \times 10^6$ cells analyzed; Figure 4 B), from which we prepared genomic DNA for the determination of erbB2 CN. This analysis was performed in all of the other five samples analyzed, with similar results (data not shown). The peripheral blood of the healthy controls was analyzed in the same way, but did not show any cell population positive for CK8, CK18 and CK19 and EpCAM, and negative for CD45 (data not shown) . The analyses of this DNA extracted from these CTCs of the EC patients showed that the erbB2 CN was in the same range (CN >2) as for the DNA isolated from the plasma of the same (six) EC patients (Figure 4C). The comparison bet ween the CN values from plasma and from CTCs was showed in table 2.

**ErbB2 protein expression in tumor tissue**

We additionally performed immunohistochemistry analysis for erbB2 protein staining (in parallel sections) in 13 esophageal primary tumor tissue samples, four of the tissues of which were included in the plasma DNA dataset previously analyzed. Some tissue staining examples are shown in the additional file, in figure 6. Strong expression of erbB2 was showed for the case code 1 patient (score +++), with intermediate expression of erbB2 for the case code 2 patient (score ++) and low expression for the case code 3 patient (score +) (Figure 6A). Control experiments showed weak staining of erbB in proliferative epithelial tissues of healthy esophageal mucosa (Figure 6B). The table 4 shows the quantization of DNA extracted from plasma of patients and controls.

Table 4

| CN patients code | DNA from plasma concentration (ng/μl) | Healthy control subjects code | DNA from plasma concentration (ng/μl) |
|---|---|---|---|
| P1 | 144 | e10 | 40 |
| P2 | 159 | e12 | 34 |
| P3 | 144 | e13 | 27 |
| P4 | 95 | e14 | 14 |
| P5 | 239 | e2 | 7 |
| P6 | 201 | e20 | 78 |
| P7 | 178 | e22 | 64 |
| P8 | 197 | e23 | 8 |
| P9 | 265 | e3 | 33 |
| P10 | 155 | e40 | 45 |
| P11 | 137 | e41 | 67 |
| P12 | 298 | e46 | 86 |
| P13 | 301 | e48 | 53 |
| P14 | 164 | e49 | 37 |
| P15 | 234 | e5 | 10 |
| P16 | 209 | e51 | 24 |
| P17 | 309 | e52 | 39 |
| P18 | 258 | e53 | 28 |
| P19 | 123 | e55 | 20 |
| P20 | 143 | e56 | 7 |
| P22 | 276 | e57 | 81 |
| P24 | 302 | e6 | 90 |
| P25 | 175 | e7 | 38 |
| P26 | 274 | e8 | 66 |
| P27 | 128 | f3 | 89 |
| P28 | 132 | f4 | 36 |
| P29 | 298 | f5 | 73 |
| P30 | 304 | f6 | 82 |
| P31 | 309 | e1 | 94 |
| P32 | 259 | e15 | 13 |

(continued)

| P33 | 155 | e18 | 56 |
|---|---|---|---|
| P34 | 235 | e19 | 58 |
| P35 | 354 | e21 | 83 |
| P36 | 125 | e24 | 27 |
| P37 | 249 | | |
| P39 | 187 | | |
| P40 | 299 | | |
| P41 | 310 | | |
| P42 | 322 | | |
| P43 | 182 | | |
| P44 | 221 | | |

**Bibliografia**

1. Ferlay J, Autier P, Boniol M, Heanue M, Colombet M, Boyle P: Estimates of the cancer incidence and mortality in Europe in 2006. Annals of oncology : official journal of the European Society for Medical Oncology / ESMO 2007, 18(3):581-592.

2. Lagergren J: Adenocarcinoma of oesophagus: what exactly is the size of the problem and who is at risk? Gut 2005, 54 Suppl 1:i1-5.

3. Ancona E, Rampado S, Cassaro M, Battaglia G, Ruol A, Castoro C, Portale G, Cavallin F, Rugge M: Prediction of lymph node status in superficial esophageal carcinoma. Annals of surgical oncology 2008, 15(11):3278-3288.

4. Bird-Lieberman EL, Fitzgerald RC: Early diagnosis of oesophageal cancer. British journal of cancer 2009, 101 (1):1-6.

5. Endo M, Kawano T: Detection and classification of early squamous cell esophageal cancer. Diseases of the esophagus : official journal of the International Society for Diseases of the Esophagus / ISDE 1997, 10(3):155-158.

6. Koppert LB, Wijnhoven BP, van Dekken H, Tilanus HW, Dinjens WN: The molecular biology of esophageal adenocarcinoma. Journal of surgical oncology 2005, 92(3):169-190.

7. Crew KD, Neugut AI: Epidemiology of upper gastrointestinal malignancies. Seminars in oncology 2004, 31 (4): 450-464.

8. Vallbohmer D, Lenz HJ: Predictive and prognostic molecular markers in outcome of esophageal cancer. Diseases of the esophagus : official journal of the International Society for Diseases of the Esophagus / ISDE 2006, 19(6): 425-432.

9. Slamon DJ, Leyland-Jones B, Shak S, Fuchs H, Paton V, Bajamonde A, Fleming T, Eiermann W, Wolter J, Pegram M et al: Use of chemotherapy plus a monoclonal antibody against HER2 for metastatic breast cancer that overexpresses HER2. The New England journal of medicine 2001, 344(11):783-792.

10. Reichelt U, Duesedau P, Tsourlakis M, Quaas A, Link BC, Schurr PG, Kaifi JT, Gros SJ, Yekebas EF, Marx A et al: Frequent homogeneous HER-2 amplification in primary and metastatic adenocarcinoma of the esophagus. Modern pathology : an official journal of the United States and Canadian Academy of Pathology, Inc 2007, 20(1): 120-129.

11. Stoecklein NH, Hosch SB, Bezler M, Stern F, Hartmann CH, Vay C, Siegmund A, Scheunemann P, Schurr P, Knoefel WT et al: Direct genetic analysis of single disseminated cancer cells for prediction of outcome and therapy selection in esophageal cancer. Cancer cell 2008, 13(5):441-453.

12. Chiang PW, Beer DG, Wei WL, Orringer MB, Kurnit DM: Detection of erbB-2 amplifications in tumors and sera from esophageal carcinoma patients. Clinical cancer research : an official journal of the American Association for Cancer Research 1999, 5(6):1381-1386.

13. Folkman J: Angiogenesis in cancer, vascular, rheumatoid and other disease. Nature medicine 1995, 1(1):27-31.

14. He Y, Karpanen T, Alitalo K: Role of lymphangiogenic factors in tumor metastasis. Biochimica et biophysica acta 2004, 1654(1):3-12.

15. Risau W: Mechanisms of angiogenesis. Nature 1997, 386(6626):671-674.

16. Ferrara N: VEGF and the quest for tumour angiogenesis factors. Nature reviews Cancer 2002, 2(10):795-803.

17. Mustonen T, Alitalo K: Endothelial receptor tyrosine kinases involved in angiogenesis. The Journal of cell biology 1995, 129(4):895-898.

18. Kitadai Y, Amioka T, Haruma K, Tanaka S, Yoshihara M, Sumii K, Matsutani N, Yasui W, Chayama K: Clinicopathological significance of vascular endothelial growth factor (VEGF)-C in human esophageal squamous cell carcinomas. International journal of cancer Journal international du cancer 2001, 93(5):662-666.

19. von Rahden BH, Stein HJ, Puhringer F, Koch I, Langer R, Piontek G, Siewert JR, Hofler H, Sarbia M: Coexpression of cyclooxygenases (COX-1, COX-2) and vascular endothelial growth factors (VEGF-A, VEGF-C) in esophageal adenocarcinoma. Cancer research 2005, 65(12):5038-5044.

20. Krzystek-Korpacka M, Matusiewicz M, Diakowska D, Grabowski K, Blachut K, Banas T: Up-regulation of VEGF-C secreted by cancer cells and not VEGF-A correlates with clinical evaluation of lymph node metastasis in esophageal squamous cell carcinoma (ESCC). Cancer letters 2007, 249(2):171-177.

21. Andolfatto S, Namour F, Garnier AL, Chabot F, Gueant JL, Aimone-Gastin I: Genomic DNA extraction from small amounts of serum to be used for alpha1-antitrypsin genotype analysis. The European respiratory journal : official journal of the European Society for Clinical Respiratory Physiology 2003, 21(2):215-219.

22. Castaldo G, Tomaiuolo R, Sanduzzi A, Bocchino ML, Ponticiello A, Barra E, Vitale D, Bariffi F, Sacchetti L, Salvatore F: Lung cancer metastatic cells detected in blood by reverse transcriptase-polymerase chain reaction and dot-blot analysis. Journal of clinical oncology : official journal of the American Society of Clinical Oncology 1997, 15 (11):3388-3393.

23. Hoon DS, Spugnardi M, Kuo C, Huang SK, Morton DL, Taback B: Profiling epigenetic inactivation of tumor suppressor genes in tumors and plasma from cutaneous melanoma patients. Oncogene 2004, 23(22):4014-4022.

24. Koike M, Hibi K, Kasai Y, Ito K, Akiyama S, Nakao A: Molecular detection of circulating esophageal squamous cell cancer cells in the peripheral blood. Clinical cancer research : an official journal of the American Association for Cancer Research 2002, 8(9):2879-2882.

25. Koyanagi K, Mori T, O'Day SJ, Martinez SR, Wang HJ, Hoon DS: Association of circulating tumor cells with serum tumor-related methylated DNA in peripheral blood of melanoma patients. Cancer research 2006, 66(12): 6111-6117.

26. Pathak AK, Bhutani M, Kumar S, Mohan A, Guleria R: Circulating cell-free DNA in plasma/serum of lung cancer patients as a potential screening and prognostic tool. Clinical chemistry 2006, 52(10):1833-1842.

27. Taback B, Fujiwara Y, Wang HJ, Foshag LJ, Morton DL, Hoon DS: Prognostic significance of circulating microsatellite markers in the plasma of melanoma patients. Cancer research 2001, 61 (15):5723-5726.

28. Klein CA, Stoecklein NH: Lessons from an aggressive cancer: evolutionary dynamics in esophageal carcinoma. Cancer research 2009, 69(13):5285-5288.

29. Bang YJ, Van Cutsem E, Feyereislova A, Chung HC, Shen L, Sawaki A, Lordick F, Ohtsu A, Omuro Y, Satoh T et al: Trastuzumab in combination with chemotherapy versus chemotherapy alone for treatment of HER2-positive advanced gastric or gastro-oesophageal junction cancer (ToGA): a phase 3, open-label, randomised controlled trial. Lancet 2010, 376(9742):687-697.

30. Shih CH, Ozawa S, Ando N, Ueda M, Kitajima M: Vascular endothelial growth factor expression predicts outcome and lymph node metastasis in squamous cell carcinoma of the esophagus. Clinical cancer research : an official journal of the American Association for Cancer Research 2000, 6(3):1161-1168.

31. Banki F, Yacoub WN, Hagen JA, Mason RJ, Ayazi S, DeMeester SR, Lipham JC, Danenberg K, Danenberg P, DeMeester TR: Plasma DNA is more reliable than carcinoembryonic antigen for diagnosis of recurrent esophageal cancer. Journal of the American College of Surgeons 2008, 207(1):30-35.

32. Boni L, Cassinotti E, Canziani M, Dionigi G, Rovera F, Dionigi R: Free circulating DNA as possible tumour marker in colorectal cancer. Surgical oncology 2007, 16 Suppl 1:S29-31.

33. Diehl F, Schmidt K, Durkee KH, Moore KJ, Goodman SN, Shuber AP, Kinzler KW, Vogelstein B: Analysis of mutations in DNA isolated from plasma and stool of colorectal cancer patients. Gastroenterology 2008, 135(2): 489-498.

34. Maheswaran S, Sequist LV, Nagrath S, Ulkus L, Brannigan B, Collura CV, Inserra E, Diederichs S, Iafrate AJ, Bell DW et al: Detection of mutations in EGFR in circulating lung-cancer cells. The New England journal of medicine 2008, 359(4):366-377.

35. Perego RA, Corizzato M, Brambilla P, Ferrero S, Bianchi C, Fasoli E, Signorini S, Torsello B, Invernizzi L, Bombelli S et al: Concentration and microsatellite status of plasma DNA for monitoring patients with renal carcinoma. European journal of cancer 2008, 44(7):1039-1047.

36. Tomita H, Ichikawa D, Ikoma D, Sai S, Tani N, Ikoma H, Fujiwara H, Kikuchi S, Okamoto K, Ochiai T et al: Quantification of circulating plasma DNA fragments as tumor markers in patients with esophageal cancer. Anticancer research 2007, 27(4C):2737-2741.

37. Van der Auwera I, Elst HJ, Van Laere SJ, Maes H, Huget P, van Dam P, Van Marck EA, Vermeulen PB, Dirix LY: The presence of circulating total DNA and methylated genes is associated with circulating tumour cells in blood from breast cancer patients. British journal of cancer 2009, 100(8):1277-1286.

38. Mimura K, Kono K, Hanawa M, Mitsui F, Sugai H, Miyagawa N, Ooi A, Fujii H: Frequencies of HER-2/neu expression and gene amplification in patients with oesophageal squamous cell carcinoma. British journal of cancer 2005, 92(7):1253-1260.

39. Sato-Kuwabara Y, Neves JI, Fregnani JH, Sallum RA, Soares FA: Evaluation of gene amplification and protein expression of HER-2/neu in esophageal squamous cell carcinoma using Fluorescence in situ Hybridization (FISH) and immunohistochemistry. BMC cancer 2009, 9:6.

40. Bresalier RS: Barrett's Esophagus and esophageal adenocarcinoma. Annual review of medicine 2009, 60: 221-231.

41. Reid BJ, Li X, Galipeau PC, Vaughan TL: Barrett's oesophagus and oesophageal adenocarcinoma: time for a new synthesis. Nature reviews Cancer 2010, 10(2):87-101.

42. Hongo M, Nagasaki Y, Shoji T: Epidemiology of esophageal cancer: Orient to Occident. Effects of chronology, geography and ethnicity. Journal of gastroenterology and hepatology 2009, 24(5):729-735.

43. Livak KJ, Schmittgen TD: Analysis of relative gene expression data using real-time quantitative PCR and the 2 (-Delta Delta C(T)) Method. Methods 2001, 25(4):402-408.

44. Ponchel F, Toomes C, Bransfield K, Leong FT, Douglas SH, Field SL, Bell SM, Combaret V, Puisieux A, Mighell AJ et al: Real-time PCR based on SYBR-Green I fluorescence: an alternative to the TaqMan assay for a relative quantification of gene rearrangements, gene amplifications and micro gene deletions. BMC biotechnology 2003, 3:18.

45. Yuan JS, Reed A, Chen F, Stewart CN, Jr.: Statistical analysis of real-time PCR data. BMC bioinformatics 2006, 7:85.

46. Dhanasekaran S, Doherty TM, Kenneth J, Group TBTS: Comparison of different standards for real-time PCR-based absolute quantification. Journal of immunological methods 2010, 354(1-2):34-39.

47. Godornes C, Leader BT, Molini BJ, Centurion-Lara A, Lukehart SA: Quantitation of rabbit cytokine mRNA by real-time RT-PCR. Cytokine 2007, 38(1):1-7.

48. Lieto E, Ferraraccio F, Orditura M, Castellano P, Mura AL, Pinto M, Zamboli A, De Vita F, Galizia G: Expression of vascular endothelial growth factor (VEGF) and epidermal growth factor receptor (EGFR) is an independent prognostic indicator of worse outcome in gastric cancer patients. Annals of surgical oncology 2008, 15(1):69-79.

49. Kallergi G, Markomanolaki H, Giannoukaraki V, Papadaki MA, Strati A, Lianidou ES, Georgoulias V, Mavroudis D, Agelaki S: Hypoxia-inducible factor-1alpha and vascular endothelial growth factor expression in circulating tumor cells of breast cancer patients. Breast cancer research : BCR 2009, 11(6): R84.

**Claims**

1. Intron nucleotide sequence of erbB2 gene for use as circulating tumor cells biomarker of a tumor chosen in the group consisting of colon cancer, esophageal cancer, gastric cancer, breast cancer, lung cancer, medulloblastoma, prostate cancer, lymphoma, melanoma, glioblastoma, pancreas carcinoma, sarcoma, hepatocarcinoma, said intronic sequence consisting of: CGCTGGGCAGGTATGCAgggctgacgtagtgcctttgtggcagcagTTTCGTGG CACACATTCTGC (SEQ ID No: 1) or TATGCAGGGCTGACGTAGTGCctttgtggcAGCAGTTTCGTGGCACACA TT (SEQ ID No: 2) or CGCUGGGCAGGUAUGCAGGGCUGACGUAGUGCCUUUGUGGCAG CAGUUUCGUGGCACACAUUCUGC (SEQ ID No: 13).

2. Method *in vitro* for detecting circulating tumor cells of a tumor chosen in the group consisting of colon cancer, esophageal cancer, gastric cancer, breast cancer, lung cancer, medulloblastoma, prostate cancer, lymphoma, melanoma, glioblastoma, pancreas carcinoma, sarcoma, hepatocarcinoma, said method being **characterized by** the detection of copy number variation of erbB2 gene in cells of a biological sample in comparison to the copy number of said gene in cells of a healthy control by means of TaqMan method using the following primer couple and probe for the amplification of SEQ ID No: 1:

   erbB2 _for: CGCTGGGCAGGTATGCA (SEQ ID No: 3)
   erbB2 _rev : GCAGAATGTGTGCCACGAAA (SEQ ID No: 4)
   probe erbB2: CTGACGTAGTGCCTTTGTGGCAGCA (SEQ ID No: 5)
   and a primer couple and probe for the amplification of a normalizing gene chosen in the group consisting β-actin ID: 60, GAPDH Gene ID: 2597, UBB Gene ID: 7314, TUBA6 Gene ID: 84790, RPS13 Gene ID: 6207, NACA Gene ID: 4666, B2M Gene ID: 567, TBP Gene ID: 6908.

3. Method according to claim 2, wherein the primer couple and probe for the amplification of a normalizing gene consist of the following primer couple and probe for the amplification of β-actin gene:

   β-actin _for : CCCAGCCACACCACAAAGTC (SEQ ID No: 6)
   β-actin _rev : CCAGTTGAATAAAAGTGCACACCTT(SEQ ID No:7)
   probe β-actin : CACTTGGCCTCATTTT (SEQ ID No: 8)

4. Method *in vitro* for detecting circulating tumor cells of a tumor chosen in the group consisting of colon cancer, esophageal cancer, gastric cancer, breast cancer, lung cancer, medulloblastoma, prostate cancer, lymphoma, melanoma, glioblastoma, pancreas carcinoma, sarcoma, hepatocarcinoma, said method being **characterized by** the detection of copy number variation of erbB2 gene in cells of a biological sample in comparison to the copy number of said gene in cells of a healthy control by means of Syber method using the following primer couple for the amplification of SEQ ID No: 2:

   erbB2 S F: TATGCAGGGCTGACGTAGTGC (SEQ ID No: 9)
   erbB2 S R: AATGTGTGCCACGAAACTGCT (SEQ ID No: 10)
   and a primer couple for the amplification of a normalizing gene chosen in the group consisting of β-actin gene ID: 60, GAPDH Gene ID: 2597, UBB Gene ID: 7314, TUBA6 Gene ID: 84790, RPS13 Gene ID: 6207, NACA Gene ID: 4666, B2M Gene ID: 567, TBP Gene ID: 6908.

5. Method according to claim 4, wherein the primer couple for the amplification of a normalizing gene consists of the following primer couple for the amplification of β-actin gene:

   β-actin F: CCTCACCCTGAAGTACCCCA (SEQ ID No: 11)
   β-actin R: TCGTCCCAGTTGGTGACGAT (SEQ ID No: 12)

6. Method *in vitro* for detecting circulating tumor cells of a tumor chosen in the group consisting of colon cancer, esophageal cancer, gastric cancer, breast cancer, lung cancer, medulloblastoma, prostate cancer, lymphoma, melanoma, glioblastoma, pancreas carcinoma, sarcoma, hepatocarcinoma, said method being **characterized by** the detection of different erbB2 expression in heterogeneous RNA of biological sample cells in comparison to erbB2 expression in heterogeneous RNA of healthy control cells by means of TaqMan method using the following primer couple and probe for the amplification of SEQ ID No: 13:

   erbB2 RNA_for: CGCUGGGCAGGUAUGCA (SEQ ID No: 14)

erbB2 RNA rev : GCAGAAUGUGUGCCACGAAA (SEQ ID No:15)
probe erbB2 RNA: CUGACGUAGUGCCUUUGUGGCAGCA (SEQ ID No:16)
and a primer couple and probe for the amplification of a normalizing gene chosen in the group consisting of β-actin gene ID: 60; GAPDH Gene ID: 2597, UBB Gene ID: 7314, TUBA6 Gene ID: 84790, RPS13 Gene ID: 6207, NACA Gene ID: 4666, B2M Gene ID: 567, TBP Gene ID: 6908.

7. Method according to anyone of the claims 2-6, wherein the biological sample is chosen among serum/plasma, peripheral blood, medullary blood, urine, faeces, cephalorrachidian fluid, ascitic fluid, gastric juice, seminal liquid, saliva, preural liquid, bile of patient.

8. Kit for the *in vitro* detection of circulating tumor cells of a tumor chosen in the group consisting of colon cancer, esophageal cancer, gastric cancer, breast cancer, lung cancer, medulloblastoma, prostate cancer, lymphoma, melanoma, glioblastoma, pancreas carcinoma, sarcoma, hepatocarcinoma, said kit comprising or consisting of the following primers and probes for the detection of copy number variation of erbB2 gene in cells of a biological sample in comparison to the copy number of said gene in cells of a healthy control by means of TaqMan method:

erbB2 _for: CGCTGGGCAGGTATGCA (SEQ ID No: 3)
erbB2 _rev : GCAGAATGTGTGCCACGAAA (SEQ ID No: 4)
probe erbB2: CTGACGTAGTGCCTTTGTGGCAGCA (SEQ ID No: 5) and a primer couple and probe for the amplification of a normalizing gene chosen in the group consisting of β-actin gene ID: 60, GAPDH Gene ID: 2597, UBB Gene ID: 7314, TUBA6 Gene ID: 84790, RPS13 Gene ID: 6207, NACA Gene ID: 4666, B2M Gene ID: 567, TBP Gene ID: 6908.

9. Kit according to claim 8, wherein the primer couple and probe for the amplification of a normalizing gene consist of the following primer couple and probe for the amplification of β-actin gene:

β-actin _for : CCCAGCCACACCACAAAGTC (SEQ ID No: 6)
β-actin _rev : CCAGTTGAATAAAAGTGCACACCTT(SEQ ID No:7)
probe β-actin : CACTTGGCCTCATTTT (SEQ ID No: 8)

10. Kit for the *in vitro* detection of circulating tumor cells of a tumor chosen in the group consisting of colon cancer, esophageal cancer, gastric cancer, breast cancer, lung cancer, medulloblastoma, prostate cancer, lymphoma, melanoma, glioblastoma, pancreas carcinoma, sarcoma, hepatocarcinoma, said kit comprising or consisting of the following primers couple for the detection of copy number variation of erbB2 gene in cells of a biological sample in comparison to the copy number of said gene in cells of a healthy control by means of Syber method:

erbB2 S F: TATGCAGGGCTGACGTAGTGC (SEQ ID No:9)
erbB2 S R: AATGTGTGCCACGAAACTGCT (SEQ ID No: 10)
and a primer couple for the amplification of a normalizing gene chosen in the group consisting of β-actin gene ID: 60, GAPDH Gene ID: 2597, UBB Gene ID: 7314, TUBA6 Gene ID: 84790, RPS13 Gene ID: 6207, NACA Gene ID: 4666, B2M Gene ID: 567, TBP Gene ID: 6908.

11. Kit according to claim 10, wherein the primer couple for the amplification of a normalizing gene consists of the following primer couple for the amplification of β-actin gene:

β-actin F: CCTCACCCTGAAGTACCCCA (SEQ ID No: 11)
β-actin R: TCGTCCCAGTTGGTGACGAT (SEQ ID No: 12)

12. Kit for *in vitro* detection of circulating tumor cells of a tumor chosen in the group consisting of colon cancer, esophageal cancer, gastric cancer, breast cancer, lung cancer, medulloblastoma, prostate cancer, lymphoma, melanoma, glioblastoma, pancreas carcinoma, sarcoma, hepatocarcinoma, said kit comprising or consisting of the following primer couple and probe for the detection of different erbB2 expression in heterogeneous RNA of biological sample cells in comparison to erbB2 expression in heterogeneous RNA of healthy control cells by means of TaqMan method:

erbB2 RNA_for: CGCUGGGCAGGUAUGCA (SEQ ID No: 14)
erbB2 RNA rev : GCAGAAUGUGUGCCACGAAA (SEQ ID No:15)
probe erbB2 RNA: CUGACGUAGUGCCUUUGUGGCAGCA (SEQ ID No:16)
and a primer couple and probe for the amplification of a normalizing gene chosen in the group consisting of β-actin gene ID: 60, GAPDH Gene ID: 2597, UBB Gene ID: 7314, TUBA6 Gene ID: 84790, RPS13 Gene ID: 6207, NACA Gene ID: 4666, B2M Gene ID: 567, TBP Gene ID: 6908.

13. Intron nucleotide sequence of erbB2 gene consisting of:

CGCTGGGCAGGTATGCAgggctgacgtagtgcctttgtggcagcagTTTCG TGGCACACATTCTGC (SEQ ID No: 1) or
TATGCAGGGCTGACGTAGTGCctttgtggcAGCAGTTTCGTGGCACACA TT (SEQ ID No: 2) or

CGCUGGGCAGGUAUGCAGGGCUGACGUAGUGCCUUUGUG
GCAGCAGUUUCGUGGCACACAUUCUGC (SEQ ID No: 13).

*Fig.1*

Fig.2

Follow-up months

Fig.3

Fig.4

Fig.5

A

ErbB2

B

ErbB2

*Fig.6*

Fig.7

*Fig.8*

Europäisches
Patentamt

European
Patent Office

Office européen
des brevets

# EUROPEAN SEARCH REPORT

**Application Number**

EP 12 42 5061

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHIANG ET AL: "Detection of erbB-2 amplifications in tumors and sera from esophageal carcinoma patients.", CLINICAL CANCER RESEARCH, vol. 5, no. 6, 1 June 1999 (1999-06-01), pages 1381-1386, XP055008747, ISSN: 1078-0432 * page 1384 - page 1385, paragraph 1; table 2 * | 1-13 | INV. C12Q1/68 |
| X | CAO S. ET AL: "Quantitative determination of HER2 expression by confocal microscopy assay in CTCs of breast cancer", ONCOLOGY REPORTS, vol. 23, no. 2, 28 December 2009 (2009-12-28), XP055008799, ISSN: 1021-335X, DOI: 10.3892/or_00000651 * page 425, right-hand column - page 426; tables 2, 3 * | 1-13 | |
| X | EP 2 199 798 A1 (UNIV BRUXELLES [BE]) 23 June 2010 (2010-06-23) * claims 7,8; example 1; table 1 * | 1-13 | TECHNICAL FIELDS SEARCHED (IPC)  C12Q |
| X | EP 1 772 521 A1 (ONCOSCORE AG [CH]) 11 April 2007 (2007-04-11) * sequence 38 * | 1,8-13 | |

-/--

The present search report has been drawn up for all claims

| Place of search | Date of completion of the search | Examiner |
|---|---|---|
| The Hague | 20 June 2012 | Santagati, Fabio |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**EUROPEAN SEARCH REPORT**

Application Number

EP 12 42 5061

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | NIKOLAS H. STOECKLEIN ET AL: "Direct Genetic Analysis of Single Disseminated Cancer Cells for Prediction of Outcome and Therapy Selection in Esophageal Cancer", CANCER CELL, vol. 13, no. 5, 1 May 2008 (2008-05-01), pages 441-453, XP055009123, ISSN: 1535-6108, DOI: 10.1016/j.ccr.2008.04.005 * figure 3; table 2 * | 1-13 | |
| A | BRIEN ET AL: "HER-2/neu gene amplification by FISH predicts poor survival in Barrett's esophagus-associated adenocarcinoma", HUMAN PATHOLOGY, SAUNDERS, PHILADELPHIA, PA, US, vol. 31, no. 1, 1 January 2000 (2000-01-01), pages 35-39, XP005047623, ISSN: 0046-8177 * the whole document * | 1-13 | TECHNICAL FIELDS SEARCHED (IPC) |

The present search report has been drawn up for all claims

| Place of search | Date of completion of the search | Examiner |
|---|---|---|
| The Hague | 20 June 2012 | Santagati, Fabio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 12 42 5061

This annex lists the patent family members relating to the patent documents cited in  the above-mentioned European search report.
The members are as contained in the European Patent Office EDP file on
The European Patent Office is in no way liable for these particulars which are merely given for the purpose of information.

20-06-2012

| Patent document cited in search report | | Publication date | Patent family member(s) | | Publication date |
|---|---|---|---|---|---|
| EP 2199798 | A1 | 23-06-2010 | EP<br>WO | 2199798 A1<br>2010070124 A1 | 23-06-2010<br>24-06-2010 |
| EP 1772521 | A1 | 11-04-2007 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Estimates of the cancer incidence and mortality in Europe. **FERLAY J ; AUTIER P ; BONIOL M ; HEANUE M ; COLOMBET M ; BOYLE P.** Annals of oncology : official journal of the European Society for Medical Oncology / ESMO. 2006, vol. 18, 581-592

- **LAGERGREN J.** Adenocarcinoma of oesophagus: what exactly is the size of the problem and who is at risk?. *Gut,* 2005, vol. 54 (1), i1-5

- Prediction of lymph node status in superficial esophageal carcinoma. **ANCONA E ; RAMPADO S ; CASSARO M ; BATTAGLIA G ; RUOL A ; CASTORO C ; PORTALE G ; CAVALLIN F ; RUGGE M.** Annals of surgical oncology. 2008, vol. 15, 3278-3288

- **BIRD-LIEBERMAN EL ; FITZGERALD RC.** Early diagnosis of oesophageal cancer. *British journal of cancer,* 2009, vol. 101 (1), 1-6

- **ENDO M ; KAWANO T.** Detection and classification of early squamous cell esophageal cancer. *Diseases of the esophagus : official journal of the International Society for Diseases of the Esophagus / ISDE,* 1997, vol. 10 (3), 155-158

- **KOPPERT LB ; WIJNHOVEN BP ; VAN DEKKEN H ; TILANUS HW ; DINJENS WN.** The molecular biology of esophageal adenocarcinoma. *Journal of surgical oncology,* 2005, vol. 92 (3), 169-190

- **CREW KD ; NEUGUT AI.** Epidemiology of upper gastrointestinal malignancies. *Seminars in oncology,* 2004, vol. 31 (4), 450-464

- **VALLBOHMER D ; LENZ HJ.** Predictive and prognostic molecular markers in outcome of esophageal cancer. *Diseases of the esophagus : official journal of the International Society for Diseases of the Esophagus / ISDE,* 2006, vol. 19 (6), 425-432

- **SLAMON DJ ; LEYLAND-JONES B ; SHAK S ; FUCHS H ; PATON V ; BAJAMONDE A ; FLEMING T ; EIERMANN W ; WOLTER J ; PEGRAM M et al.** Use of chemotherapy plus a monoclonal antibody against HER2 for metastatic breast cancer that overexpresses HER2. *The New England journal of medicine,* 2001, vol. 344 (11), 783-792

- **REICHELT U ; DUESEDAU P ; TSOURLAKIS M ; QUAAS A ; LINK BC ; SCHURR PG ; KAIFI JT ; GROS SJ ; YEKEBAS EF ; MARX A et al.** Frequent homogeneous HER-2 amplification in primary and metastatic adenocarcinoma of the esophagus. *Modern pathology : an official journal of the United States and Canadian Academy of Pathology, Inc,* 2007, vol. 20 (1), 120-129

- **STOECKLEIN NH ; HOSCH SB ; BEZLER M ; STERN F ; HARTMANN CH ; VAY C ; SIEGMUND A ; SCHEUNEMANN P ; SCHURR P ; KNOEFEL WT et al.** Direct genetic analysis of single disseminated cancer cells for prediction of outcome and therapy selection in esophageal cancer. *Cancer cell,* 2008, vol. 13 (5), 441-453

- **CHIANG PW ; BEER DG ; WEI WL ; ORRINGER MB ; KURNIT DM.** Detection of erbB-2 amplifications in tumors and sera from esophageal carcinoma patients. *Clinical cancer research : an official journal of the American Association for Cancer Research,* 1999, vol. 5 (6), 1381-1386

- **FOLKMAN J.** Angiogenesis in cancer, vascular, rheumatoid and other disease. *Nature medicine,* 1995, vol. 1 (1), 27-31

- **HE Y ; KARPANEN T ; ALITALO K.** Role of lymphangiogenic factors in tumor metastasis. *Biochimica et biophysica acta,* 2004, vol. 1654 (1), 3-12

- **RISAU W.** Mechanisms of angiogenesis. *Nature,* 1997, vol. 386 (6626), 671-674

- **FERRARA N.** VEGF and the quest for tumour angiogenesis factors. *Nature reviews Cancer,* 2002, vol. 2 (10), 795-803

- **MUSTONEN T ; ALITALO K.** Endothelial receptor tyrosine kinases involved in angiogenesis. *The Journal of cell biology,* 1995, vol. 129 (4), 895-898

- **KITADAI Y ; AMIOKA T ; HARUMA K ; TANAKA S ; YOSHIHARA M ; SUMII K ; MATSUTANI N ; YASUI W ; CHAYAMA K.** Clinicopathological significance of vascular endothelial growth factor (VEGF)-C in human esophageal squamous cell carcinomas. *International journal of cancer Journal international du cancer,* 2001, vol. 93 (5), 662-666

- **VON RAHDEN BH ; STEIN HJ ; PUHRINGER F ; KOCH I ; LANGER R ; PIONTEK G ; SIEWERT JR ; HOFLER H ; SARBIA M.** Coexpression of cyclooxygenases (COX-1, COX-2) and vascular endothelial growth factors (VEGF-A, VEGF-C) in esophageal adenocarcinoma. *Cancer research,* 2005, vol. 65 (12), 5038-5044

- **KRZYSTEK-KORPACKA M ; MATUSIEWICZ M ; DIAKOWSKA D ; GRABOWSKI K ; BLACHUT K ; BANAS T.** Up-regulation of VEGF-C secreted by cancer cells and not VEGF-A correlates with clinical evaluation of lymph node metastasis in esophageal squamous cell carcinoma (ESCC). *Cancer letters,* 2007, vol. 249 (2), 171-177

- **ANDOLFATTO S ; NAMOUR F ; GARNIER AL ; CHABOT F ; GUEANT JL ; AIMONE-GASTIN I.** Genomic DNA extraction from small amounts of serum to be used for alpha1-antitrypsin genotype analysis. *The European respiratory journal : official journal of the European Society for Clinical Respiratory Physiology,* 2003, vol. 21 (2), 215-219
- **CASTALDO G ; TOMAIUOLO R ; SANDUZZI A ; BOCCHINO ML ; PONTICIELLO A ; BARRA E ; VITALE D ; BARIFFI F ; SACCHETTI L ; SALVATORE F.** Lung cancer metastatic cells detected in blood by reverse transcriptase-polymerase chain reaction and dot-blot analysis. *Journal of clinical oncology : official journal of the American Society of Clinical Oncology,* 1997, vol. 15 (11), 3388-3393
- **HOON DS ; SPUGNARDI M ; KUO C ; HUANG SK ; MORTON DL ; TABACK B.** Profiling epigenetic inactivation of tumor suppressor genes in tumors and plasma from cutaneous melanoma patients. *Oncogene,* 2004, vol. 23 (22), 4014-4022
- **KOIKE M ; HIBI K ; KASAI Y ; ITO K ; AKIYAMA S ; NAKAO A.** Molecular detection of circulating esophageal squamous cell cancer cells in the peripheral blood. *Clinical cancer research : an official journal of the American Association for Cancer Research,* 2002, vol. 8 (9), 2879-2882
- **KOYANAGI K ; MORI T ; O'DAY SJ ; MARTINEZ SR ; WANG HJ ; HOON DS.** Association of circulating tumor cells with serum tumor-related methylated DNA in peripheral blood of melanoma patients. *Cancer research,* 2006, vol. 66 (12), 6111-6117
- **PATHAK AK ; BHUTANI M ; KUMAR S ; MOHAN A ; GULERIA R.** Circulating cell-free DNA in plasma/serum of lung cancer patients as a potential screening and prognostic tool. *Clinical chemistry,* 2006, vol. 52 (10), 1833-1842
- **TABACK B ; FUJIWARA Y ; WANG HJ ; FOSHAG LJ ; MORTON DL ; HOON DS.** Prognostic significance of circulating microsatellite markers in the plasma of melanoma patients. *Cancer research,* 2001, vol. 61 (15), 5723-5726
- **KLEIN CA ; STOECKLEIN NH.** Lessons from an aggressive cancer: evolutionary dynamics in esophageal carcinoma. *Cancer research,* 2009, vol. 69 (13), 5285-5288
- **BANG YJ ; VAN CUTSEM E ; FEYEREISLOVA A ; CHUNG HC ; SHEN L ; SAWAKI A ; LORDICK F ; OHTSU A ; OMURO Y ; SATOH T et al.** Trastuzumab in combination with chemotherapy versus chemotherapy alone for treatment of HER2-positive advanced gastric or gastro-oesophageal junction cancer (ToGA): a phase 3, open-label, randomised controlled trial. *Lancet,* 2010, vol. 376 (9742), 687-697
- **SHIH CH ; OZAWA S ; ANDO N ; UEDA M ; KITAJIMA M.** Vascular endothelial growth factor expression predicts outcome and lymph node metastasis in squamous cell carcinoma of the esophagus. *Clinical cancer research : an official journal of the American Association for Cancer Research,* 2000, vol. 6 (3), 1161-1168
- **BANKI F ; YACOUB WN ; HAGEN JA ; MASON RJ ; AYAZI S ; DEMEESTER SR ; LIPHAM JC ; DANENBERG K ; DANENBERG P ; DEMEESTER TR.** Plasma DNA is more reliable than carcinoembryonic antigen for diagnosis of recurrent esophageal cancer. *Journal of the American College of Surgeons,* 2008, vol. 207 (1), 30-35
- **BONI L ; CASSINOTTI E ; CANZIANI M ; DIONIGI G ; ROVERA F ; DIONIGI R.** Free circulating DNA as possible tumour marker in colorectal cancer. *Surgical oncology,* 2007, vol. 16 (1), S29-31
- **DIEHL F ; SCHMIDT K ; DURKEE KH ; MOORE KJ ; GOODMAN SN ; SHUBER AP ; KINZLER KW ; VOGELSTEIN B.** Analysis of mutations in DNA isolated from plasma and stool of colorectal cancer patients. *Gastroenterology,* 2008, vol. 135 (2), 489-498
- **MAHESWARAN S ; SEQUIST LV ; NAGRATH S ; ULKUS L ; BRANNIGAN B ; COLLURA CV ; INSERRA E ; IEDERICHS S ; LAFRATE AJ ; BELL DW et al.** Detection of mutations in EGFR in circulating lung-cancer cells. *The New England journal of medicine,* 2008, vol. 359 (4), 366-377
- **PEREGO RA ; CORIZZATO M ; BRAMBILLA P ; FERRERO S ; BIANCHI C ; FASOLI E ; SIGNORINI S ; TORSELLO B ; INVERNIZZI L ; BOMBELLI S et al.** Concentration and microsatellite status of plasma DNA for monitoring patients with renal carcinoma. *European journal of cancer,* 2008, vol. 44 (7), 1039-1047
- **TOMITA H ; ICHIKAWA D ; IKOMA D ; SAI S ; TANI N ; IKOMA H ; FUJIWARA H ; KIKUCHI S ; OKAMOTO K ; OCHIAI T et al.** Quantification of circulating plasma DNA fragments as tumor markers in patients with esophageal cancer. *Anticancer research,* 2007, vol. 27 (4C), 2737-2741
- **VAN DER AUWERA I ; ELST HJ ; VAN LAERE SJ ; MAES H ; HUGET P ; VAN DAM P ; VAN MARCK EA ; VERMEULEN PB ; DIRIX LY.** The presence of circulating total DNA and methylated genes is associated with circulating tumour cells in blood from breast cancer patients. *British journal of cancer,* 2009, vol. 100 (8), 1277-1286
- **MIMURA K ; KONO K ; HANAWA M ; MITSUI F ; SUGAI H ; MIYAGAWA N ; OOI A ; FUJII H.** Frequencies of HER-2/neu expression and gene amplification in patients with oesophageal squamous cell carcinoma. *British journal of cancer,* 2005, vol. 92 (7), 1253-1260

- **SATO-KUWABARA Y ; NEVES JI ; FREGNANI JH ; SALLUM RA ; SOARES FA.** Evaluation of gene amplification and protein expression of HER-2/neu in esophageal squamous cell carcinoma using Fluorescence in situ Hybridization (FISH) and immunohistochemistry. *BMC cancer,* 2009, vol. 9, 6
- **BRESALIER RS.** Barrett's Esophagus and esophageal adenocarcinoma. *Annual review of medicine,* 2009, vol. 60, 221-231
- **REID BJ ; LI X ; GALIPEAU PC ; VAUGHAN TL.** Barrett's oesophagus and oesophageal adenocarcinoma: time for a new synthesis. *Nature reviews Cancer,* 2010, vol. 10 (2), 87-101
- **HONGO M ; NAGASAKI Y ; SHOJI T.** Epidemiology of esophageal cancer: Orient to Occident. Effects of chronology, geography and ethnicity. *Journal of gastroenterology and hepatology,* 2009, vol. 24 (5), 729-735
- **LIVAK KJ ; SCHMITTGEN TD.** Analysis of relative gene expression data using real-time quantitative PCR and the 2(-Delta Delta C(T)) Method. *Methods,* 2001, vol. 25 (4), 402-408
- **PONCHEL F ; TOOMES C ; BRANSFIELD K ; LEONG FT ; DOUGLAS SH ; FIELD SL ; BELL SM ; COMBARET V ; PUISIEUX A ; MIGHELL AJ et al.** Real-time PCR based on SYBR-Green I fluorescence: an alternative to the TaqMan assay for a relative quantification of gene rearrangements, gene amplifications and micro gene deletions. *BMC biotechnology,* 2003, vol. 3, 18
- **YUAN JS ; REED A ; CHEN F ; STEWART CN, JR.** Statistical analysis of real-time PCR data. *BMC bioinformatics,* 2006, vol. 7, 85
- **DHANASEKARAN S ; DOHERTY TM ; KENNETH J.** Group TBTS: Comparison of different standards for real-time PCR-based absolute quantification. *Journal of immunological methods,* 2010, vol. 354 (1-2), 34-39
- **GODORNES C ; LEADER BT ; MOLINI BJ ; CENTURION-LARA A ; LUKEHART SA.** Quantitation of rabbit cytokine mRNA by real-time RT-PCR. *Cytokine,* 2007, vol. 38 (1), 1-7
- Expression of vascular endothelial growth factor (VEGF) and epidermal growth factor receptor (EGFR) is an independent prognostic indicator of worse outcome in gastric cancer patients. **LIETO E ; FERRARACCIO F ; ORDITURA M ; CASTELLANO P ; MURA AL ; PINTO M ; ZAMBOLI A ; DE VITA F ; GALIZIA G.** Annals of surgical oncology. 2008, vol. 15, 69-79
- **KALLERGI G ; MARKOMANOLAKI H ; GIANNOU-KARAKI V ; PAPADAKI MA ; STRATI A ; LIANIDOU ES ; GEORGOULIAS V ; MAVROUDIS D ; AGELAKI S.** Hypoxia-inducible factor-1alpha and vascular endothelial growth factor expression in circulating tumor cells of breast cancer patients. Breast cancer research :. *BCR,* 2009, vol. 11 (6), R84

SEQUENCE LISTING

<110> Zollo, Massimo

<120> Biomarker for detecting circulating tumor cells and related detecting methods and kit

<130> BE28625

<150> RM2011A000149
<151> 2011-03-25

<160> 16

<170> PatentIn version 3.5

<210> 1
<211> 66
<212> DNA
<213> Homo sapiens

<400> 1
cgctgggcag gtatgcaggg ctgacgtagt gcctttgtgg cagcagtttc gtggcacaca        60

ttctgc        66


<210> 2
<211> 51
<212> DNA
<213> Homo sapiens

<400> 2
tatgcagggc tgacgtagtg cctttgtggc agcagtttcg tggcacacat t        51


<210> 3
<211> 17
<212> DNA
<213> artificial

<220>
<223> erbB2 gene forward primer

<400> 3
cgctgggcag gtatgca        17


<210> 4
<211> 20
<212> DNA
<213> artificial

<220>
<223> erbB2 gene reverse primer

<400> 4
gcagaatgtg tgccacgaaa        20


<210> 5
<211> 25
<212> DNA
<213> artificial

<220>
<223> erbB2 gene probe

<400> 5
ctgacgtagt gcctttgtgg cagca                                    25


<210> 6
<211> 20
<212> DNA
<213> artificial

<220>
<223> beta actin gene forward primer

<400> 6
cccagccaca ccacaaagtc                                          20


<210> 7
<211> 25
<212> DNA
<213> artificial

<220>
<223> beta actin gene reverse primer

<400> 7
ccagttgaat aaaagtgcac acctt                                    25


<210> 8
<211> 16
<212> DNA
<213> artificial

<220>
<223> beta actin gene probe

<400> 8
cacttggcct catttt                                              16


<210> 9
<211> 21
<212> DNA
<213> artificial

<220>
<223> erbB2 gene forward primer

<400> 9
tatgcagggc tgacgtagtg c                                        21


<210> 10
<211> 21
<212> DNA
<213> artificial

<220>
<223> erbB2 gene reverse primer

<400> 10
aatgtgtgcc acgaaactgc t                                        21


<210> 11
<211> 20
<212> DNA
<213> artificial

<220>
<223> beta actin gene forward primer

<400> 11
cctcaccctg aagtacccca                                                      20


<210> 12
<211> 20
<212> DNA
<213> artificial

<220>
<223> beta actin gene reverse primer

<400> 12
tcgtcccagt tggtgacgat                                                      20


<210> 13
<211> 66
<212> RNA
<213> Homo sapiens

<400> 13
cgcugggcag guaugcaggg cugacguagu gccuuugugg cagcaguuuc guggcacaca          60

uucugc                                                                     66


<210> 14
<211> 17
<212> RNA
<213> artificial

<220>
<223> erbB2 RNA forward primer

<400> 14
cgcugggcag guaugca                                                         17


<210> 15
<211> 20
<212> RNA
<213> artificial

<220>
<223> erbB2 RNA reverse primer

<400> 15
gcagaaugug ugccacgaaa                                                      20


<210> 16
<211> 25
<212> RNA
<213> artificial

<220>
<223> erbB2 RNA probe

<400> 16
cugacguagu gccuuugugg cagca                                                25